# EUROPEAN PATENT APPLICATION

(11) **EP 3 444 011 A1**
(43) Date of publication of application: **20.02.2019**
(21) Application number: 18178387.9
(22) Date of filing: 11.05.2015
(51) Int. Cl.: A61P 1/16, A61P 35/00, A61K 31/167

(54) **TREATMENT OF THE COMPLICATIONS OF CHRONIC LIVER DISEASE WITH EMRICASAN**

(30) Priority: 12.05.2014 US 201461992169 P
(62) Divisional of application: 15726430.0
(71) Applicant: Conatus Pharmaceuticals, Inc., San Diego, CA 92127 (US)
(72) Inventor: Spada, Alfred P., Carlsbad, CA California 92009 (US)
(74) Representative: Jones Day

(57) **Abstract**

Provided herein is a compound for use in a method of treating or preventing a complication of a chronic liver disease, wherein the compound is: 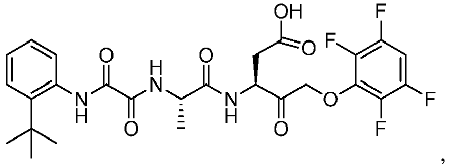 or a pharmaceutically acceptable salt thereof.

## Description

### 1. CROSS REFERENCE TO RELATED APPLICATIONS

This application claims the benefit of the priority of U.S. Provisional Application No. 61/992,169, filed May 12, 2014, the disclosure of which is incorporated herein by reference in its entirety.

### 2. FIELD

Provided herein are methods of treating certain complications of chronic liver disease by administering a caspase inhibitor.

### 3. BACKGROUND

Complications arising from chronic liver disease affect a large patient population and is associated with a high degree of morbidity and mortality. Portal hypertension is an important and common clinical condition that is a complication of chronic liver disease and is defined as elevated blood pressure in the liver as determined by hepatic venous pressure gradient (HVPG) of greater than 5 mmHg. Normal HVPG is considered to be between 3 and 5 mmHg. While all portal blood pressure above 5 mmHg is considered abnormal, an HVPG measurement of greater than 10 mmHg has been shown to be highly predictive of the development of serious debilitating and potentially life threatening consequences. The clinical outcome of patients with portal pressures of 10 mmHg or above include: the development of esophageal varices, ascites, hepatic encephalopathy, variceal hemorrhage and hepatocellular carcinoma. Portal hypertension can arise from a variety of clinical conditions that prevent normal blood flow and normal hemodynamics both in and to the liver. Conditions such as thrombosis in the splenic or portal vein, Budd-Chiari syndrome or for example schistosomiasis cause portal hypertension. In these conditions, blood vessels are blocked and cause an increase in blood pressure. Portal hypertension is also a complication of liver cirrhosis, or scarring of the liver. Scarring of the liver in cirrhosis increases liver stiffness and results in portal hypertension and compromised liver function. Scarring in cirrhosis also increases death of cells in the liver. One form of cell death in the liver is through a process known as apoptosis. Another form of cell death in the liver is through inflammation. Cirrhosis is the end-stage clinical complication of any chronic liver disease.

Advanced chronic liver disease affects a large patient population and is associated with a high degree of morbidity and mortality. In 2009, chronic liver disease was the 4^{th} leading cause of death in the United States among persons between the ages of 45 to 54 (see, Asrani et al. Hepatology, 2013;145:375-382). In view of the fact that the complications of chronic liver disease affects a large patient population, there is a strong need to provide new and effective pharmaceutical agents for these patients.

### 4. SUMMARY

The methods, compounds, pharmaceutical compositions and articles of manufacture provided herein are characterized by a variety of component ingredients, steps of preparation, and steps of execution and associated biophysical, physical, biochemical or chemical parameters. As would be apparent to those of skill in the art, the methods provided herein can include any and all permutations and combinations of the compounds, compositions, articles of manufacture and associated ingredients, steps and/or parameters as described below.

In one aspect, provided herein are methods for treating portal hypertension by a caspase inhibitor. In some embodiments, the portal hypertension is a consequence of chronic liver disease. Caspase inhibitors as known to and understood by one of skill in the art are contemplated herein. Exemplary compounds for use in the methods are described elsewhere herein. Also provided are pharmaceutical compositions for use in the methods.

In certain embodiments, the methods provided herein include treatment of portal hypertension resulting from chronic liver disease. In one embodiment, chronic liver disease is caused by toxins, including alcohol, some drugs, and the abnormal build-up of normal substances in the blood. In another embodiment, chronic liver disease is caused by infection or by an autoimmune disorder. In certain embodiments, the exact cause of the chronic liver disease is not known. In certain embodiments, the chronic liver disease include, but is not limited to viral infection, fatty liver, cirrhosis, primary biliary cirrhosis, primary sclerosing cholangitis, Budd-Chiari syndrome and alpha 1-antitrypsin deficiency.

In one embodiment, the chronic liver disease includes, but is not limited to cirrhosis, liver fibrosis, non-alcoholic fatty liver disease (NAFLD), non-alcoholic steatohepatitis (NASH), hepatitis, including viral and alcoholic hepatitis, primary biliary cirrhosis (PBC) and primary sclerosing cholangitis (PSC).

In certain embodiments, provided are methods for the treatment of patients who have failed therapy for portal hypertension arising from chronic liver disease. In one embodiment, the methods provided herein reduce portal hypertension associated with chronic liver diseases. In one embodiment, the methods provided herein lower elevated levels of liver enzymes, such as elevated levels of ALT (alanine aminotransferase) and AST (aspartate aminotransferase) levels. In one embodiment, the methods provided herein-improve liver function associated with chronic liver diseases.

Also provided are caspase inhibitors for use in the methods provided herein. In one embodiment, the caspase inhibitor compound for use in the methods provided herein is selected from: 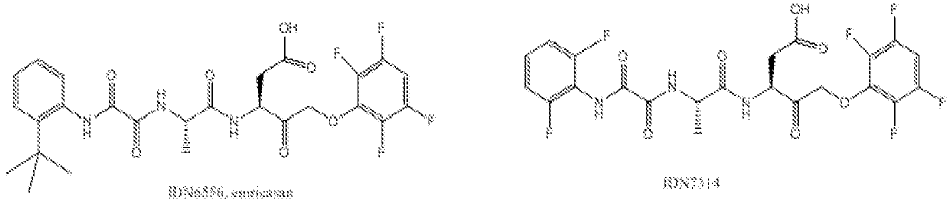 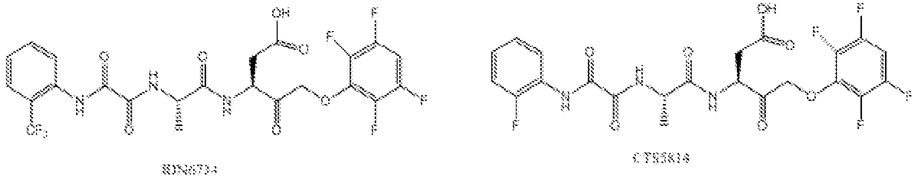 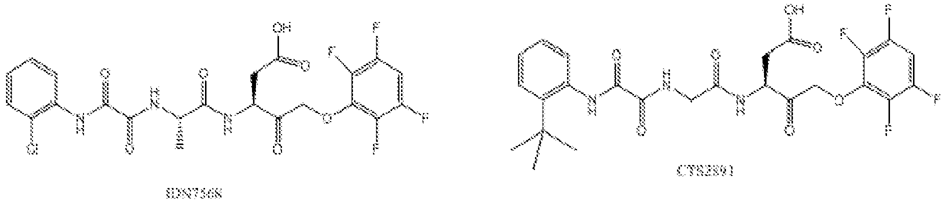 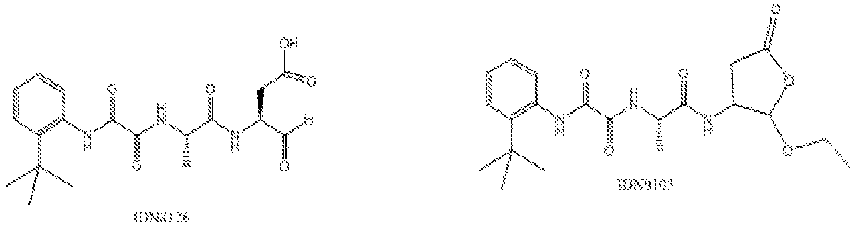 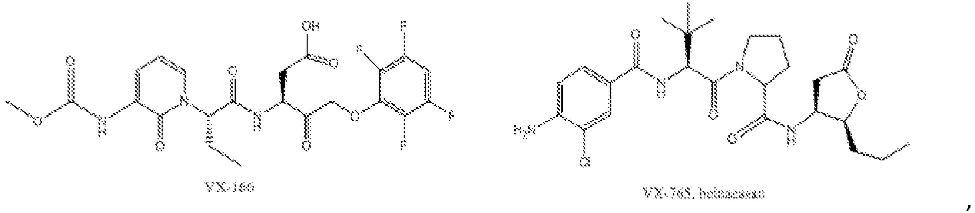 or a pharmaceutically acceptable derivative thereof. In one embodiment, the pharmaceutically acceptable derivative is a pharmaceutically acceptable salt.

In one embodiment, the caspase inhibitor for use in the methods provided herein is 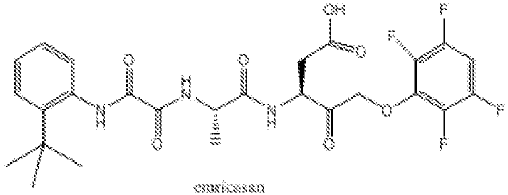 or a pharmaceutically acceptable derivative thereof. In one embodiment, the pharmaceutically acceptable derivative is a pharmaceutically acceptable salt.

In some embodiments, more than one caspase inhibitor can be used sequentially or simultaneously in the methods provided herein.

Also provided are pharmaceutical compositions containing therapeutically effective amounts of the compounds provided herein and a pharmaceutically acceptable carrier, wherein the pharmaceutical compositions are useful in the prevention, treatment, or amelioration of one or more of the symptoms of liver diseases. In some embodiments, the symptom is portal hypertension. In other embodiments, the liver disease is a chronic liver disease selected from cirrhosis, liver fibrosis, non-alcoholic fatty liver disease (NAFLD), non-alcoholic steatohepatitis (NASH), hepatitis, including viral and alcoholic hepatitis, primary biliary cirrhosis (PBC) and primary sclerosing cholangitis (PSC). In yet other embodiments, the portal hypertension is a consequence of chronic liver disease. In some embodiments, the chronic liver disease is cirrhosis.

Further provided is an article of manufacture containing packaging material, the compounds or pharmaceutically acceptable derivatives thereof provided herein, which is used for treatment, prevention or amelioration of one or more symptoms associated with a liver disease, and a label that indicates that compounds or pharmaceutically acceptable derivatives thereof are used for treatment, prevention or amelioration of one or more symptoms of a liver disease. In some embodiments, the symptom is portal hypertension. In other embodiments, the liver disease is a chronic liver disease selected from cirrhosis, liver fibrosis, non-alcoholic fatty liver disease (NAFLD), non-alcoholic steatohepatitis (NASH), hepatitis, including viral and alcoholic hepatitis, primary biliary cirrhosis (PBC) and primary sclerosing cholangitis (PSC). In yet other embodiments, the portal hypertension is a consequence of chronic liver disease. In some embodiments, the chronic liver disease is cirrhosis.

### 5. DETAILED DESCRIPTION OF THE PREFERRED EMBODIMENTS

### 5.1. Definitions

Unless defined otherwise, all technical and scientific terms used herein have the same meaning as is commonly understood by one of ordinary skill in the art. All patents, applications, published applications and other publications are incorporated by reference in their entirety. In the event that there are a plurality of definitions for a term herein, those in this section prevail unless stated otherwise.

The singular forms "a," "an," and "the" include plural references, unless the context clearly dictates otherwise.

As used herein "subject" is an animal, such as a mammal, including human, such as a patient.

As used herein, biological activity refers to the *in vivo* activities of a compound or physiological responses that result upon *in vivo* administration of a compound, composition or other mixture. Biological activity, thus, encompasses therapeutic effects and pharmacokinetic behavior of such compounds, compositions and mixtures. Biological activities can be observed in *in vitro* systems designed to test for such activities.

As used herein, pharmaceutically acceptable derivatives of a compound include salts, esters, acetals, ketals, orthoesters, hemiacetals, hemiketals, acids, bases, solvates, hydrates or prodrugs thereof. Such derivatives may be readily prepared by those of skill in this art using known methods for such derivatization. The compounds produced may be administered to animals or humans without substantial toxic effects and either are pharmaceutically active or are prodrugs. Pharmaceutically acceptable salts include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamine and other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc; and inorganic salts, such as but not limited to, sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates, mesylates, and fumarates. Pharmaceutically acceptable esters include, but are not limited to, alkyl, alkenyl, alkynyl, aryl, aralkyl, and cycloalkyl esters of acidic groups, including, but not limited to, carboxylic acids, phosphoric acids, phosphinic acids, sulfonic acids, sulfinic acids and boronic acids. Pharmaceutically acceptable solvates and hydrates are complexes of a compound with one or more solvent or water molecules, or 1 to about 100, or 1 to about 10, or one to about 2, 3 or 4, solvent or water molecules.

As used herein, treatment means any manner in which one or more of the symptoms of a disease or disorder are ameliorated or otherwise beneficially altered. Treatment also encompasses any pharmaceutical use of the compositions herein, such as use for treating a liver disease.

As used herein, amelioration of the symptoms of a particular disorder by administration of a particular compound or pharmaceutical composition refers to any lessening, whether permanent or temporary, lasting or transient that can be attributed to or associated with administration of the composition.

As used herein, "complication" refers to a condition that develops in association with a condition or disease. The complication can be as a direct result caused by the condition or disease, or can be associated with the existence of the primary condition or disease. For example, portal hypertension can be a complication of chronic liver disease. Additionally, cirrhosis can be a complication of chronic liver disease. In some embodiments, the complications of a disease can be manifested as a symptom and, in those instances, the two terms are used interchangeably herein.

As used herein, and unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

It is to be understood that the compounds provided herein may contain chiral centers. Such chiral centers may be of either the (R) or (S) configuration, or may be a mixture thereof. Thus, the compounds provided herein may be enantiomerically pure, or be stereoisomeric or diastereomeric mixtures. As such, one of skill in the art will recognize that administration of a compound in its (R) form is equivalent, for compounds that undergo epimerization *in vivo,* to administration of the compound in its (S) form.

As used herein, substantially pure means sufficiently homogeneous to appear free of readily detectable impurities as determined by standard methods of analysis, such as thin layer chromatography (TLC), gel electrophoresis, high performance liquid chromatography (HPLC) and mass spectrometry (MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound. The instant disclosure is meant to include all such possible isomers, as well as, their racemic and optically pure forms. Optically active (+) and (-), (R)- and (S)-, or (D)- and (L)-isomers may be prepared using chiral synthons or chiral reagents, or resolved using conventional techniques, such as reverse phase HPLC. When the compounds described herein contain olefinic double bonds or other centers of geometric asymmetry, and unless specified otherwise, it is intended that the compounds include both E and Z geometric isomers. Likewise, all tautomeric forms are also intended to be included.

In certain embodiments, the compound used in the methods provided herein is "stereochemically pure." A stereochemically pure compound or has a level of stereochemical purity that would be recognized as "pure" by those of skill in the art. In certain embodiments, "stereochemically pure" designates a compound that is substantially free of alternate isomers. In particular embodiments, the compound is 85%, 90%, 91%, 92%, 93%, 94%, 95%, 96%, 97%, 98%, 99%, 99.5% or 99.9% free of other isomers.

As used herein, "therapy for liver disease" refers to a treatment with any medication known, available in the market and being developed for the treatment of liver disease. For example, therapy of portal hypertension refers to treatment of the patient with drugs available in the market for the treatment of portal hypertension. Several exemplary drugs are described in the section on "Combination Therapy" *infra.*

As used herein, "therapy for chronic liver disease" refers to a treatment with any medication known, available in the market and being developed for the treatment of chronic liver disease. For example, therapy for portal hypertension refers to treatment of the patient with drugs available in the market for portal hypertension treatment. Several exemplary drugs are described in the section on "Combination Therapy" *infra.*

As used herein, "treatment" means any manner in which one or more of the symptoms of a disease or disorder are ameliorated or otherwise beneficially altered. Treatment also encompasses any pharmaceutical use of the compositions herein, such as use for treating a liver disease.

As used herein, "mitigate," means the reduction or elimination of symptoms. Mitigate also means the reduction of severity or the delayed progression of disease or being otherwise beneficially altered.

As used herein, amelioration of the symptoms of a particular disorder by administration of a particular compound or pharmaceutical composition refers to any lessening, whether permanent or temporary, lasting or transient that can be attributed to or associated with administration of the composition.

As used herein, "patients who have failed therapy" refers to the patient population described elsewhere herein and includes patients that have previously been treated for a liver disease with any of the drugs currently available in the market and either did not respond to the therapy (used synonymously herein with "failed therapy"), could not tolerate the therapy or for whom the therapy was medically contraindicated. For example, response to treatment for portal hypertension in patients whose portal pressure is between 6 and 10 mm Hg is defined as a reduction of 10 percent or greater in portal pressure from baseline portal pressure value in a patient with portal hypertension. Patients not achieving a reduction of 10 percent or greater in portal pressure from baseline portal pressure value are patients that are medically classified as patients that did not respond to therapy. In patients with portal pressure greater than 12 mmHg, response is defined as a reduction below 12 mmHg or a 20 percent or greater reduction from the patient's baseline portal pressure. Patients not achieving the above mentioned are considered treatment failures.

Therapy for portal hypertension is contraindicated for some patients. For example, treatment of portal hypertension with non-selective beta blockers in patients who also have asthma, chronic obstructive pulmonary disease (COPD) or sinus bradycardia and partial AV block (prolongation of electrical conduction in the heart) is medically contraindicated.

Therapy for portal hypertension is not tolerated by some patients due to the development of undesirable side effects as understood by those with skill in the art. Inability to tolerate therapy can include, but is not limited to, weakness, shortness of breath and fatigue.

As used herein, the term "in combination" refers to the use of more than one therapies *(e.g.,* a caspase inhibitor and other agents). The use of the term "in combination" does not restrict the order in which therapies *(e.g.,* a caspase inhibitor and other agents) are administered to a subject with a disorder. A first therapy *(e.g.,* a caspase inhibitor and other agents) can be administered prior to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of other therapy *(e.g.,* a caspase inhibitor and other agents) to a subject with a disorder.

As used herein, the term "synergistic" refers to a combination of a caspase inhibitor with another agent, which is more effective than the additive effects of the administration of the two compounds as monotherapies. A synergistic effect of a combination of therapies *(e.g.,* a caspase inhibitor and another agent) permits the use of lower dosages of one or more of the therapies and/or less frequent administration of the therapies to a subject with a disorder. The ability to utilize lower dosages of a therapy *(e.g.,* a caspase inhibitor and another agent) and/or to administer the therapy less frequently reduces the toxicity associated with the administration of the therapy to a subject without reducing the efficacy of the therapy in the prevention or treatment of a disorder. In addition, a synergistic effect can result in improved efficacy of agents in the prevention or treatment of a disorder. Finally, a synergistic effect of a combination of therapies *(e.g.,* a caspase inhibitor and another agent) may avoid or reduce adverse or unwanted side effects associated with the use of either therapy alone.

### 5.2. Compounds for use in the methods

Several caspase inhibitors that can be used in the methods provided herein have been reported in the literature. Certain exemplary caspase inhibitors for use in the methods are described by Linton in Current Topics in Medicinal Chemistry, (2005) 5: 1-20; and Linton et al. in J. Med. Chem., 2005, 11, 295-322 295, U.S. patent no. 7,351,702; 7,410,956; 7,443,790; 7,553,852; 7,652,153; 7,612,091; 7,807,659; 7,857,712; 7,960,415; 8,071,618; 7,074,782; 7,053,057; 6,689,784; 6,632,962; 6,559,304; 6,201,118; 6,800,619, 6,197,750; 6,544,951; 6,790,989; 7,053,056; 7,183,260; 7,692,038. and International application nos. WO 2006/017295; WO 2005/021516; WO 04/002961; WO 02/085899; WO 02/094263 and WO 01/094351. The contents of these references are hereby incorporated by reference in their entireties.

In one embodiment, the caspase inhibitor for use in the methods provided herein is selected from 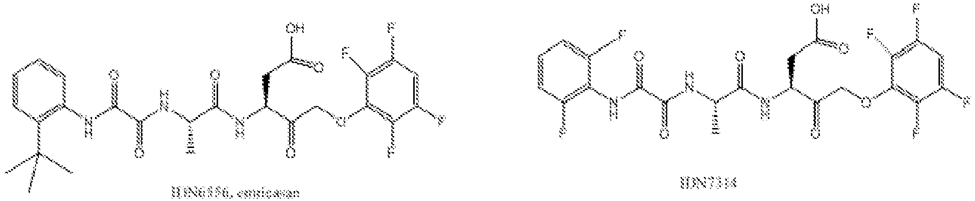 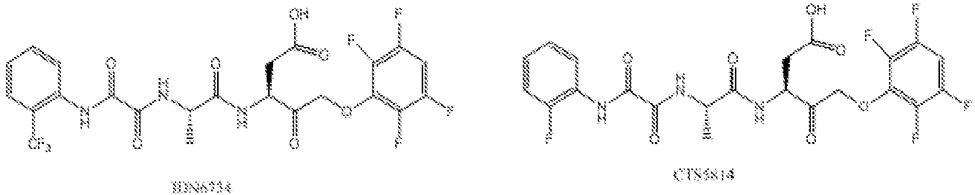 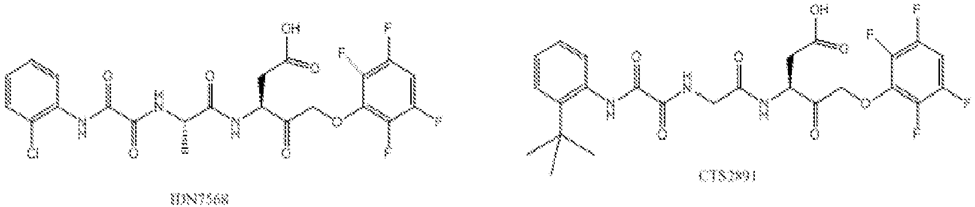 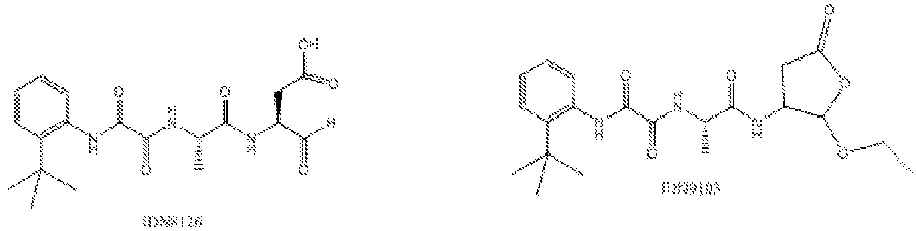 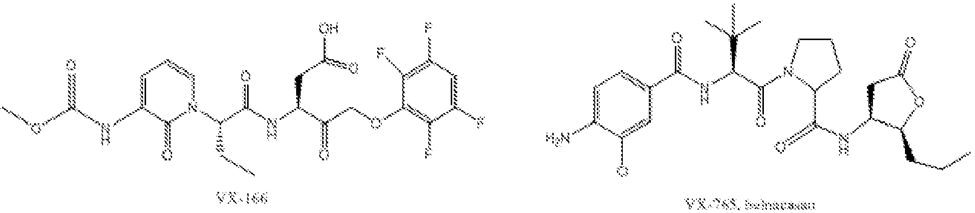 or a pharmaceutically acceptable derivative thereof. In one embodiment, the pharmaceutically acceptable derivative is a pharmaceutically acceptable salt.

In one embodiment, the caspase inhibitor for use in the methods provided herein is selected from 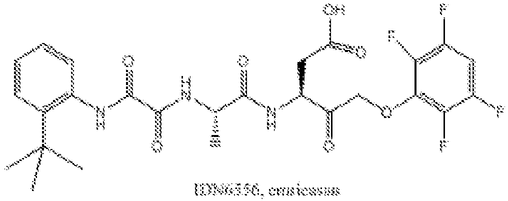 or a pharmaceutically acceptable derivative thereof. In one embodiment, the pharmaceutically acceptable derivative is a pharmaceutically acceptable salt.

In some embodiments, more than one caspase inhibitor can be used sequentially or simultaneously in the methods provided herein.

In certain embodiments, the compounds described herein have efficacy in models of liver disease following oral administration of from 0.001 - 1000 mg/Kg. In certain embodiments, the compounds described herein have efficacy in models of liver disease following oral administration of from 0.01 - 100 mg/Kg.

### 5.3. Methods of treatment

In certain embodiments, the methods provided herein include treatment of chronic liver disease. In one embodiment, the methods are for treatment of complications of chronic liver disease. In one embodiment, the methods are for reducing portal hypertension associated with chronic liver disease. In one embodiment, the methods are for the reduction of cirrhosis. Without being bound to any particular theory, it is believed that the caspase inhibitors used in the methods provided herein act by inhibiting apoptosis and/or inflammation and the generation and signaling of vasoactive cytokines that affect the liver and intestinal vascular system that leads to portal hypertension.

In one embodiment, the chronic liver disease is a disorder that results from an injury to the liver. In one embodiment, injury to the liver is caused by toxins, including alcohol, some drugs, and the abnormal build-up of normal substances in the blood. In another embodiment, the chronic liver injury is caused by an infection or by an autoimmune disorder. In certain embodiments, the exact cause of the injury is not known.

In one embodiment, the chronic liver disease includes, but is not limited to cirrhosis, liver fibrosis, non-alcoholic fatty liver disease (NAFLD), non-alcoholic steatohepatitis (NASH), hepatitis, including viral and alcoholic hepatitis, primary biliary cirrhosis (PBC) and primary sclerosing cholangitis (PSC). In one embodiment, the chronic liver disease is manifested by conditions known to those of skill in the art including, but not limited to, portal hypertension, raised liver enzymes (*e.g*., ALT and AST), alkaline phosphatase (ALP), elevated bilirubin, pathological evidence of cirrhosis, steatosis (fatty liver) or fibrosis.

In one embodiment, chronic liver disease is manifested by conditions known to those of skill in the art including, but not limited to, raised hepatic portal pressure, raised liver enzymes (*e.g*., ALT, AST), histological evidence of liver damage and cirrhosis.

In one embodiment, the methods provided herein are for treating portal hypertension. Symptoms of portal hypertension may include but not limited to, splenomegaly, small spider-like veins in skin, gastrointestinal bleeding marked by black tarry stools or blood in the stools, ascites (build-up of fluid in the abdominal cavity), encephalopathy, spider angiomas (a central arteriole from which numerous small branching vessels radiate). Symptoms of portal hypertension can vary, depending on severity and individuals. In certain embodiments, mild portal hypertension specified as between 6 to 10 mmHg, may not exhibit any symptoms at all. Moderate levels of portal hypertension are between 10 to 12 mmHg and severe portal hypertension is classified as greater than or equal to 12 mmHg. Methods for measuring portal hypertension are well known in the art (see, Berzigotti, A. et al. Assessing portal hypertension in liver diseases, Expert Reviews Gastroenterol. Hepatol. 2013;7:141-155. and Koh, C. et al. Approach to the diagnosis of portal hypertension. Clinical Liver Disease 2012;1:133-135. and incorporated by reference herein in their entirety) In one embodiment, the clinically defined elevated level of portal pressure is reduced by at least 95%, at least 90%, at least 80%, at least 70%, at least 60%, at least 50%, at least 40%, at least 30%, at least 20%, at least 10%, at least 5%, at least 2% or at least 1%.

In certain embodiments, provided are methods for treatment of portal hypertension for patients who have failed therapy. Exemplary methods of treatment of portal hypertension are described by Bari, K et al. Treatment of portal hypertension. World J. Gastroenterology 2012;18:1166-1175. and Giannelli, V. et al. Beta-blockers in liver cirrhosis. Annal. Gastroenterology 2014; 27:20-26 incorporated by reference herein in their entirety).

In certain embodiments, the patient is a patient that discontinued therapy for portal hypertension because of one or more adverse events associated with the therapy. Approximately 15% of patients discontinue common first line therapy with non-selective beta blockers (NSBB) owing to side effects that include fatigue, weakness and shortness of breath. In certain embodiments, the patient is a patient where current therapy is not indicated. For instance, certain patients have an absolute or relative contraindication for therapy. Contraindications include but are not limited to certain cardiovascular disease conditions and various respiratory diseases. Approximately 15% of additional patients have an absolute or relative contraindication to therapy with NSBB.

In certain embodiments, provided are methods for treatment of portal hypertension with a combination of current commercially available treatments for portal hypertension and a caspase inhibitor. Exemplary methods of treatment of portal hypertension are described by Bari, K et al. Treatment of portal hypertension. World J. Gastroenterology 2012;18:1166-1175. and Giannelli, V. et al. Beta-blockers in liver cirrhosis. Annal. Gastroenterology 2014;27:20-26. incorporated by reference herein in their entirety.

In one embodiment, the methods provided herein can lower the elevated level of liver enzyme, such as ALT and AST levels. Methods for measuring the level of elevated liver enzymes are well known in the art (see, *e.g.,* Jeong S. Y. et al. Sandwich ELISA for measurement of cytosolic aspartate aminotransferase in sera from patients with liver diseases, Clin Chem., 2003; 49(5):826 9 and Burin des Roziers N. et al. A microtiter plate assay for measurement of serum alanine aminotransferase in blood donors, Transfusion., 1995; 35(4):331 4, each of which is incorporated by reference herein in its entirety). In one embodiment, the elevated level of one or more liver enzyme, such as ALT or AST, or the total amount of elevated liver enzyme is reduced by more than about 90% or more than 95%. In one embodiment, the elevated level of one or more liver enzyme, such as elevated levels of ALT or AST, or the total amount of elevated liver enzyme is reduced by at least 95%, at least 90%, at least 80%, at least 70%, at least 60%, at least 50%, at least 40%, at least 30%, at least 20%, at least 10%, at least 5%, at least 2% or at least 1%.

In one embodiment, provided herein is a method for treating cirrhosis. In some embodiments, the method for treating cirrhosis further reduces the symptoms associated with cirrhosis. In certain embodiments, symptoms of cirrhosis can include, but are not limited to, portal hypertension, abnormal nerve function, ascites (build-up of fluid in the abdominal cavity), breast enlargement in men, coughing up or vomiting blood, curling of fingers (Dupuytren contracture of the palms), gallstones, hair loss, itching, jaundice, kidney failure, liver encephalopathy, muscle loss, poor appetite, redness of palms, salivary gland enlargement in cheeks, shrinking of testes, small spider-like veins in skin, weakness, weight loss, spider angiomas (a central arteriole from which numerous small branching vessels radiate), encephalopathy, and asterixis (flapping tremor). Symptoms of cirrhosis can vary. Cirrhosis is defined as compensated or decompensated and further classified using the so-called Child-Pugh system which is well known to individuals skilled in the art. Cirrhosis patients are classified on the basis of certain clinical parameters. Child Pugh A are compensated and may display minimal obvious symptoms. Patients classified as Child Pugh B or Child Pugh C are decompensated and can exhibit outward symptoms such as ascites.

In other embodiments, causes of cirrhosis include hepatitis induced by any cause, excessive fat deposition, viruses (*e.g*., HCV and HBV), use of certain drugs, chemical exposure, bile duct obstruction, autoimmune diseases, obstruction of outflow of blood from the liver (*i.e.,* Budd-Chiari syndrome), heart and blood vessel disturbances, alpha1-antitrypsin deficiency, high blood galactose levels, high blood tyrosine levels, glycogen storage disease, diabetes, malnutrition, hereditary accumulation of too much copper (Wilson Disease) or iron (hemochromatosis). In one embodiment, the cause of cirrhosis is alcohol abuse.

In one embodiment, provided herein is a method for treating cirrhosis. In one embodiment, cirrhosis is characterized pathologically by loss of the normal microscopic lobular architecture, and nodular regeneration. Methods for measuring the extent of cirrhosis are well known in the art. For example, measurement of the existence of cirrhosis is determined by a clinical pathologist through the histological examination of liver biopsy samples taken from the liver of the cirrhotic patient.

In certain embodiments, provided are methods for treatment of cirrhosis with a combination of current commercially available or experimental treatments for portal hypertension and a caspase inhibitor. Exemplary methods of treatment of portal hypertension are described by Bari, K et al. Treatment of portal hypertension. World J. Gastroenterology 2012;18:1166-1175. and Giannelli, V. et al. Beta-blockers in liver cirrhosis. Annal. Gastroenterology 2014;27:20-26. incorporated by reference herein in their entirety. Exemplary compounds and current experimental therapies for treatment of portal hypertension include Propranolol, Nadolol, Carvedilol and analogs or derivatives thereof as understood by those of skill in the art. Exemplary compounds and current experimental therapies for treatment of cirrhosis include Simtuzumab (GS-6624) by Gilead, Sorafenib by Bayer and Onyx, Serelaxin (RLX030) by Norvartis, Timolol, NCX-1000, Terlipressin, NGM282 by NGM Biopharmaceuticals, LUM001, by Lumena Pharmaceuticals and analogs or derivatives thereof as understood by those of skill in the art.

In certain embodiments, provided are methods for treatment of cirrhosis with a combination of current commercially available or experimental treatments for portal hypertension and a caspase inhibitor. Exemplary methods of treatment of portal hypertension are described by Bari, K et al. Treatment of portal hypertension. World J. Gastroenterology 2012;18:1166-1175. and Giannelli, V. et al. Beta-blockers in liver cirrhosis. Annal. Gastroenterology 2014;27:20-26. incorporated by reference herein in their entirety. Exemplary compounds and current experimental therapies for treatment of portal hypertension include Propranolol ((RS)-1-(1-methylethylamino)-3-(1-naphthyloxy)propan-2-ol), Nadolol ((2R*,3S*)-5-{[(2R*)-3-(tert-butylamino)-2-hydroxypropyl]oxy}-1,2,3,4-tetrahydronaphthalene-2,3-diol), Carvedilol ((±)-[3-(9H-carbazol-4-yloxy)-2-hydroxypropyl][2-(2-methoxyphenoxy)ethyl]amine) and analogs or derivatives thereof as understood by those of skill in the art. Exemplary compounds and current experimental therapies for treatment of cirrhosis include monoclonal antibodies such as the humanized monoclonal antibody Simtuzumab (GS-6624, which binds to the lysyl oxidase-like 2 (LOXL2) enzyme and can act as an immunomodulator) by Gilead, Sorafenib (4-[4-[[4-chloro-3-(trifluoromethyl)phenyl]carbamoylamino] phenoxy]-N-methylpyridine-2-carboxamide) by Bayer and Onyx, hormones such as Serelaxin (RLX030, a recombinant form of human relaxin-2 represented by the sequence L-Serine, L-α-aspartyl-L-seryl-L-tryptophyl-L-methionyl-L-α-glutamyl-L-α-glutamyl-L-valylL-isoleucyl-L-lysyl-L-leucyl-L-cysteinylglycyl-L-arginyl-L-α-glutamyl-L-leucyl-L-valyl-L-arginyl-L-alanyl-L-glutaminyl-L-isoleucyl-L-alanyl-L-isoleucyl-L-cysteinylglycyl-L- methionyl-L-seryl-L-threonyl-L-tryptophyl-, cyclic (11→11'),(23 →24')-bis(disulfide) with 5-oxo-L-prolyl-L-leucyl-L-tyrosyl-L-seryl-L-alanyl-L-leucyl-L-alanyl-L-asparaginyl-L-lysyl-L- cysteinyl-L-cysteinyl-L-histidyl-L-valylglycyl-L-cysteinyl-L-threonyl-L-lysyl-L-arginyl-L- seryl-L-leucyl-L-alanyl-L-arginyl-L-phenylalanyl-L-cysteine cyclic (10'→T5')-disulfide) by Norvartis, Timolol ((S)-1-(tert-butylamino)-3-[(4-morpholin-4-yl-1,2,5-thiadiazol-3-yl)oxy]propan-2-ol), NCX-1000 (described, for example, by Fiorucci et al. in Cardiovasc Drug Rev. 2004 Summer; 22(2):135-46), Terlipressin (1-{[(4R,7S,10S,13S,16S,19R)-19-{[({[(aminoacetyl)amino]acetyl}amino)acetyl]amino}-7-(2-amino-2-oxoethyl)-10-(3-amino-3-oxopropyl)-13-benzyl-16-(4-hydroxybenzyl)-6,9,12,15,18-pentaoxo-1,2-dithia-5,8,11,14,17-pentaazacycloicosan-4-yl]carbonyl}-L-prolyl-N-(2-amino-2-oxoethyl)-L-lysinamide), NGM282, an engineered analog of fibroblast growth factor (see, for example, Rossi et al., Journal of Hepatology, Volume 60, Issue 1, Supplement, Page S533, April 2014), LUM001 (see, for example, U.S. Patent Application Nos. 20130338093; 20130109671; 20130108573 and 20130034536), and analogs or derivatives thereof as understood by those of skill in the art.

In certain embodiments, provided are methods for treatment of cirrhosis with a combination of currently experimental treatments for portal hypertension and a caspase inhibitor. Exemplary methods of treatment of portal hypertension are described by Bari, K et al. Treatment of portal hypertension. World J. Gastroenterology 2012;18:1166-1175. and Giannelli, V. et al. Beta-blockers in liver cirrhosis. Annal. Gastroenterology 2014;27:20-26 and incorporated by reference herein in its entirety.

In certain embodiments, provided herein are methods for treatment of primary biliary cirrhosis (PBC). Primary biliary cirrhosis begins with inflammation of the bile ducts inside the liver. The inflammation blocks the flow of bile out of the liver; thus, bile remains in the liver cells or spills over into the bloodstream. As inflammation spreads from the bile ducts to the rest of the liver, a latticework of scar tissue develops throughout the liver. Portal hypertension is a common complication in PBC. In one embodiment, the methods are for treatment of PBC in women aged 35 to 60. In certain embodiments, the PBC is caused by an autoimmune disorder. The methods provided herein are useful in treating one or more of the aforementioned symptoms of primary biliary cirrhosis.

In certain embodiments, provided herein are methods for treatment of primary sclerosing cholangitis (PSC). Primary sclerosing cholangitis is characterized by chronic cholestasis that is associated with chronic inflammation and apoptosis in the biliary tract in the liver. This chronic condition can lead to cirrhosis and cancer in patients. The etiology of PSC is not well understood and there is no current effective medical therapy. In one embodiment, the methods are for treatment of PSC. Portal hypertension is a common complication in PSC. In one embodiment, primary sclerosing cholangitis occurs in association with inflammatory bowel disease. The methods provided herein are useful in treating one or more of the aforementioned symptoms of primary sclerosing cholangitis.

Apoptosis occurs mainly via two signaling pathways: a death receptor mediated extrinsic pathway or a mitochondria mediated intrinsic pathway. The extrinsic pathway originates at the plasma membrane following the engagement of a family of cytokine receptors named death receptors (such as tumor necrosis factor receptor 1 (TNF-R1), Fas/CD95, and tumor necrosis factor related apoptosis inducing ligand receptors 1 and 2 (TRAIL-R1 and TRAIL-R2) by their cognate ligands (TNF-, Fas ligand (FasL)/CD95L, TRAIL). *See,* Guicciardi et al. Gut, 2005: 54, 1024-1033 and Ghavami et al., Med. Sci. Monit., 2005: 11(11): RA337-345. As shown by Mookerjee et al. in Gut 2003;52:1182-1187, TNF-α can mediate portal hypertension in patients.

As known to one of skill in the art, the cytokines interleukins 1 beta, (IL-1 β) and interleukin 18 (IL-18), mediate inflammation in the liver and are linked to chronic liver disease. Thus, prevention or suppression of inflammation in the liver is a component in the treatment of chronic liver disease. IL-1 β and IL-18 require the action of caspases to activate their individual inflammatory activities from their respective precursor proteins, pro-IL1 beta and pro-IL-18. The precursor proteins pro-IL1 beta and pro-IL-18 lack inflammatory activity. Without being bound to any particular theory, it is believed that in certain embodiments, the prevention or suppression of excessive inflammation in the liver by compounds provided herein contributes to reducing liver damage associated with chronic liver disease. As shown by Buck, M et al. in Hepatology 2014;59:1052-1059, IL1-β can mediate of portal hypertension in patients.

### 5.4. Preparation of the compounds

The compounds for use in the methods provided herein can be prepared by using routine synthetic procedures. Exemplary procedures for the preparation of caspase inhibitors used herein are described in (6,197,750; 6,544,951; 6,790,989; 7,053,056; 7,183,260; 7,692,038, and in Linton S. et al J. Med Chem. 2005;48:,6779, Ueno H. et al. Biorg. Med. Chem. Lett. 2009;19,199-102, each of which is incorporated by reference herein in its entirety) An exemplary method for preparation of emricasan is described in Example 1.

### 5.5. Formulation of pharmaceutical compositions

The pharmaceutical compositions provided herein contain therapeutically effective amounts of one or more of compounds provided herein that are useful in the prevention, treatment, or amelioration of one or more of the symptoms of liver diseases and a pharmaceutically acceptable carrier.

The compounds are formulated into suitable pharmaceutical preparations such as solutions, suspensions, tablets, dispersible tablets, pills, capsules, powders, sustained release formulations or elixirs, for oral administration or in sterile solutions or suspensions for parenteral administration, as well as transdermal patch preparation and dry powder inhalers. In one embodiment, the compounds described above are formulated into pharmaceutical compositions using techniques and procedures well known in the art (*see, e.g.,* Remington's Pharmaceutical Sciences, 20th eds., Mack Publishing, Easton PA (2000)).

In the compositions, effective concentrations of one or more compounds or pharmaceutically acceptable derivatives is (are) mixed with a suitable pharmaceutical carrier or vehicle. The compounds may be derivatized as the corresponding salts, esters, acids, bases, solvates, hydrates or prodrugs prior to formulation, as described above. The concentrations of the compounds in the compositions are effective for delivery of an amount, upon administration, that treats, prevents, or ameliorates one or more of the symptoms of liver diseases.

In one embodiment, the compositions are formulated for single dosage administration. To formulate a composition, the weight fraction of compound is dissolved, suspended, dispersed or otherwise mixed in a selected vehicle at an effective concentration such that the treated condition is relieved or ameliorated. Pharmaceutical carriers or vehicles suitable for administration of the compounds provided herein include any such carriers known to those skilled in the art to be suitable for the particular mode of administration.

In addition, the compounds may be formulated as the sole pharmaceutically active ingredient in the composition or may be combined with other active ingredients. Liposomal suspensions, including tissue-targeted liposomes, such as tumor-targeted liposomes, may also be suitable as pharmaceutically acceptable carriers. These may be prepared according to methods known to those skilled in the art. For example, liposome formulations may be prepared as known in the art. Briefly, liposomes such as multilamellar vesicles (MLV's) may be formed by drying down egg phosphatidyl choline and brain phosphatidyl serine (7:3 molar ratio) on the inside of a flask. A solution of a compound provided herein in phosphate buffered saline (PBS) lacking divalent cations is added and the flask shaken until the lipid film is dispersed. The resulting vesicles are washed to remove unencapsulated compound, pelleted by centrifugation, and then resuspended in PBS.

The active compound is included in the pharmaceutically acceptable carrier in an amount sufficient to exert a therapeutically useful effect in the absence of undesirable side effects on the patient treated. The therapeutically effective concentration may be determined empirically by testing the compounds in *in vitro* and *in vivo* systems known in the art and then extrapolated therefrom for dosages for humans.

The concentration of active compound in the pharmaceutical composition will depend on absorption, inactivation and excretion rates of the active compound, the physicochemical characteristics of the compound, the dosage schedule, and amount administered as well as other factors known to those of skill in the art. For example, the amount that is delivered is sufficient to ameliorate one or more of the symptoms of liver diseases.

In one embodiment, a therapeutically effective dosage should produce a serum concentration of an active ingredient of from about 0.1 ng/ml to about 50-100 µg/ml, from about 0.5 ng/ml to about 80 µg/ml, from about 1 ng/ml to about 60 µg/ml, from about 5 ng/ml to about 50 µg/ml, from about 5 ng/ml to about 40 µg/ml, from about 10 ng/ml to about 35 µg/ml, from about 10 ng/ml to about 25 µg/ml, from about 10 ng/ml to about 10 µg/ml, from about 25 ng/ml to about 10 µg/ml, from about 50 ng/ml to about 10 µg/ml, from about 50 ng/ml to about 5 µg/ml, from about 100 ng/ml to about 5 µg/ml, from about 200 ng/ml to about 5 µg/ml, from about 250 ng/ml to about 5 µg/ml, from about 500 ng/ml to about 5 µg/ml, from about 1 µg/ml to about 50 µg/ml, from about 0.1 ng/ml to about 5 ng/ml, from about 1 ng/ml to about 10 ng/ml or from about 1 µg/ml to about 10 µg/ml. The pharmaceutical compositions, in certain embodiments, should provide a dosage of from about 0.001 mg to about 2000 mg of compound per kilogram of body weight per day, from about 0.002 mg to about 1000 mg of compound per kilogram of body weight per day, from about 0.005 mg to about 500 mg of compound per kilogram of body weight per day, from about 0.005 mg to about 250 mg of compound per kilogram of body weight per day, from about 0.005 mg to about 200 mg of compound per kilogram of body weight per day, from about 0.005 mg to about 100 mg of compound per kilogram of body weight per day, from about 0.001 mg to about 0.005 mg of compound per kilogram of body weight per day, from about 0.01 mg to about 100 mg of compound per kilogram of body weight per day, from about 0.02 mg to about 100 mg of compound per kilogram of body weight per day, from about 0.05 mg to about 100 mg of compound per kilogram of body weight per day, from about 0.1 mg to about 100 mg of compound per kilogram of body weight per day, from about 0.5 mg to about 100 mg of compound per kilogram of body weight per day, from about 0.75 mg to about 100 mg of compound per kilogram of body weight per day, from about 1 mg to about 100 mg of compound per kilogram of body weight per day, from about 1 mg to about 10 mg of compound per kilogram of body weight per day, from about 0.001 mg to about 5 mg of compound per kilogram of body weight per day, from about 200 mg to about 2000 mg of compound per kilogram of body weight per day, or from about 10 mg to about 100 mg of compound per kilogram of body weight per day. Pharmaceutical dosage unit forms are prepared to provide from about 1 mg to about 1000 mg, from about 1 mg to about 800 mg, from about 5 mg to about 800 mg, from about 1 mg to about 100 mg, from about 1 mg to about 50 mg, from about 5 mg to about 100 mg, from about 10 mg to about 50 mg, from about 10 mg to about 100 mg, from about 25 mg to about 50 mg, and from about 10 mg to about 500 mg of the essential active ingredient or a combination of essential ingredients per dosage unit form.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

Pharmaceutically acceptable derivatives include acids, bases and esters, salts, esters, hydrates, solvates and prodrug forms. The derivative is selected such that its pharmacokinetic properties are superior to the corresponding neutral compound.

Thus, effective concentrations or amounts of one or more of the compounds described herein or pharmaceutically acceptable derivatives thereof are mixed with a suitable pharmaceutical carrier or vehicle for systemic, topical or local administration to form pharmaceutical compositions. Compounds are included in an amount effective for ameliorating one or more symptoms of, or for treating or preventing liver diseases. The concentration of active compound in the composition will depend on absorption, inactivation, excretion rates of the active compound, the dosage schedule, amount administered, particular formulation as well as other factors known to those of skill in the art.

The compositions are intended to be administered by a suitable route, including orally, parenterally, rectally, topically, locally and via nasogastric or orogastric tube. For oral administration, capsules and tablets can be used. The compositions are in liquid, semi-liquid or solid form and are formulated in a manner suitable for each route of administration. In one embodiment, modes of administration include parenteral and oral modes of administration. In certain embodiments, oral administration is contemplated.

Solutions or suspensions used for parenteral, intradermal, subcutaneous, or topical application can include any of the following components: a sterile diluent, such as water for injection, saline solution, fixed oil, polyethylene glycol, glycerine, propylene glycol, dimethyl acetamide or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parenteral preparations can be enclosed in ampules, disposable syringes or single or multiple dose vials made of glass, plastic or other suitable material.

In instances in which the compounds exhibit insufficient solubility, methods for solubilizing compounds may be used. Such methods are known to those of skill in this art, and include, but are not limited to, using cosolvents, such as dimethylsulfoxide (DMSO), using surfactants, such as TWEEN®, or dissolution in aqueous sodium bicarbonate.

Upon mixing or addition of the compound(s), the resulting mixture may be a solution, suspension, emulsion or the like. The form of the resulting mixture depends upon a number of factors, including the intended mode of administration and the solubility of the compound in the selected carrier or vehicle. The effective concentration is sufficient for ameliorating the symptoms of the disease, disorder or condition treated and may be empirically determined.

The pharmaceutical compositions are provided for administration to humans and animals in unit dosage forms, such as tablets, capsules, pills, powders, granules, sterile parenteral solutions or suspensions, and oral solutions or suspensions, and oilwater emulsions containing suitable quantities of the compounds or pharmaceutically acceptable derivatives thereof. The pharmaceutically therapeutically active compounds and derivatives thereof are formulated and administered in unitdosage forms or multipledosage forms. Unitdose forms as used herein refer to physically discrete units suitable for human and animal subjects and packaged individually as is known in the art. Each unitdose contains a predetermined quantity of the therapeutically active compound sufficient to produce the desired therapeutic effect, in association with the required pharmaceutical carrier, vehicle or diluent. Examples of unitdose forms include ampules and syringes and individually packaged tablets or capsules. Unitdose forms may be administered in fractions or multiples thereof. A multipledose form is a plurality of identical unitdosage forms packaged in a single container to be administered in segregated unitdose form. Examples of multipledose forms include vials, bottles of tablets or capsules or bottles of pints or gallons. Hence, multiple dose form is a multiple of unitdoses which are not segregated in packaging.

Sustained-release preparations can also be prepared. Suitable examples of sustained-release preparations include semipermeable matrices of solid hydrophobic polymers containing the compound provided herein, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides, copolymers of L-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated compound remain in the body for a long time, they may denature or aggregate as a result of exposure to moisture at 37 °C, resulting in a loss of biological activity and possible changes in their structure. Rational strategies can be devised for stabilization depending on the mechanism of action involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions

Dosage forms or compositions containing active ingredient in the range of 0.001% to 100% active ingredient, 0.002% to 100% active ingredient, 0.005% to 90% active ingredient, 0.01% to 100% active ingredient, 0.05% to 100% active ingredient, 0.05% to 90% active ingredient, 0.1% to 100% active ingredient, 0.1% to 1% active ingredient, 0.1% to 0.5% active ingredient, 1% to 100% active ingredient, 1% to 99% active ingredient, 1% to 98% active ingredient, 1% to 97% active ingredient, 1% to 96% active ingredient, 1% to 95% active ingredient, 5% to 95% active ingredient, 10% to 100% active ingredient, 10% to 95% active ingredient, 15% to 95% active ingredient, 20% to 95% active ingredient, 25% to 100% active ingredient, 50% to 100% active ingredient, 50% to 95% active ingredient, 60% to 95% active ingredient or 75% to 100% active ingredient, with the balance made up from nontoxic carrier may be prepared. For oral administration, a pharmaceutically acceptable nontoxic composition is formed by the incorporation of any of the normally employed excipients, such as, for example pharmaceutical grades of mannitol, lactose, starch, magnesium stearate, talcum, cellulose derivatives, sodium crosscarmellose, glucose, sucrose, magnesium carbonate or sodium saccharin. Such compositions include solutions, suspensions, tablets, capsules, powders and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparation of these compositions are known to those skilled in the art. The contemplated compositions may contain 0.001% to 100% active ingredient, in one embodiment! or 75-95% active ingredient.

The active compounds or pharmaceutically acceptable derivatives may be prepared with carriers that protect the compound against rapid elimination from the body, such as time release formulations or coatings.

The compositions may include other active compounds to obtain desired combinations of properties. The compounds provided herein, or pharmaceutically acceptable derivatives thereof as described herein, may also be advantageously administered for therapeutic or prophylactic purposes together with another pharmacological agent known in the general art to be of value in treating liver diseases. It is to be understood that such combination therapy constitutes a further aspect of the compositions and methods of treatment provided herein.

### Compositions for oral administration

Oral pharmaceutical dosage forms are either solid, gel or liquid. The solid dosage forms are tablets, capsules, granules, and bulk powders. Types of oral tablets include compressed, chewable lozenges and tablets which may be enteric coated, sugarcoated or film coated. Capsules may be hard or soft gelatin capsules, while granules and powders may be provided in non-effervescent or effervescent form with the combination of other ingredients known to those skilled in the art.

In certain embodiments, the formulations are solid dosage forms, such as capsules or tablets. The tablets, pills, capsules, troches and the like can contain any of the following ingredients, or compounds of a similar nature: a binder; a diluent; a disintegrating agent; a lubricant; a glidant; a sweetening agent; and a flavoring agent.

Examples of binders include microcrystalline cellulose, gum tragacanth, glucose solution, acacia mucilage, gelatin solution, sucrose and starch paste. Lubricants include talc, starch, magnesium or calcium stearate, lycopodium and stearic acid. Diluents include, for example, lactose, sucrose, starch, kaolin, salt, mannitol and dicalcium phosphate. Glidants include, but are not limited to, colloidal silicon dioxide. Disintegrating agents include crosscarmellose sodium, sodium starch glycolate, alginic acid, corn starch, potato starch, bentonite, methylcellulose, agar and carboxymethylcellulose. Coloring agents include, for example, any of the approved certified water soluble FD and C dyes, mixtures thereof; and water insoluble FD and C dyes suspended on alumina hydrate. Sweetening agents include sucrose, lactose, mannitol and artificial sweetening agents such as saccharin, and any number of spray dried flavors. Flavoring agents include natural flavors extracted from plants such as fruits and synthetic blends of compounds which produce a pleasant sensation, such as, but not limited to peppermint and methyl salicylate. Wetting agents include propylene glycol monostearate, sorbitan monooleate, diethylene glycol monolaurate and polyoxyethylene laural ether. Emeticcoatings include fatty acids, fats, waxes, shellac, ammoniated shellac and cellulose acetate phthalates. Film coatings include hydroxyethylcellulose, sodium carboxymethylcellulose, polyethylene glycol 4000 and cellulose acetate phthalate.

If oral administration is desired, the compound could be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The compounds can also be administered as a component of an elixir, suspension, syrup, wafer, sprinkle, chewing gum or the like. A syrup may contain, in addition to the active compounds, sucrose as a sweetening agent and certain preservatives, dyes and colorings and flavors.

The active materials can also be mixed with other active materials which do not impair the desired action, or with materials that supplement the desired action, such as antacids, H2 blockers, and diuretics. The active ingredient is a compound or pharmaceutically acceptable derivative thereof as described herein. Higher concentrations, up to about 98% by weight of the active ingredient may be included.

Pharmaceutically acceptable carriers included in tablets are binders, lubricants, diluents, disintegrating agents, coloring agents, flavoring agents, and wetting agents. Entericcoated tablets, because of the entericcoating, resist the action of stomach acid and dissolve or disintegrate in the neutral or alkaline intestines. Sugarcoated tablets are compressed tablets to which different layers of pharmaceutically acceptable substances are applied. Filmcoated tablets are compressed tablets which have been coated with a polymer or other suitable coating. Multiple compressed tablets are compressed tablets made by more than one compression cycle utilizing the pharmaceutically acceptable substances previously mentioned. Coloring agents may also be used in the above dosage forms. Flavoring and sweetening agents are used in compressed tablets, sugarcoated, multiple compressed and chewable tablets. Flavoring and sweetening agents are especially useful in the formation of chewable tablets and lozenges.

Liquid oral dosage forms include aqueous solutions, emulsions, suspensions, solutions and/or suspensions reconstituted from non-effervescent granules and effervescent preparations reconstituted from effervescent granules. Aqueous solutions include, for example, elixirs and syrups. Emulsions are either oil in-water or water in oil.

Elixirs are clear, sweetened, hydroalcoholic preparations. Pharmaceutically acceptable carriers used in elixirs include solvents. Syrups are concentrated aqueous solutions of a sugar, for example, sucrose, and may contain a preservative. An emulsion is a two phase system in which one liquid is dispersed in the form of small globules throughout another liquid. Pharmaceutically acceptable carriers used in emulsions are nonaqueous liquids, emulsifying agents and preservatives. Suspensions use pharmaceutically acceptable suspending agents and preservatives. Pharmaceutically acceptable substances used in noneffervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents are used in all of the above dosage forms.

Solvents include glycerin, sorbitol, ethyl alcohol and syrup. Examples of preservatives include glycerin, methyl and propylparaben, benzoic add, sodium benzoate and alcohol. Examples of nonaqueous liquids utilized in emulsions include mineral oil and cottonseed oil. Examples of emulsifying agents include gelatin, acacia, tragacanth, bentonite, and surfactants such as polyoxyethylene sorbitan monooleate. Suspending agents include sodium carboxymethylcellulose, pectin, tragacanth, Veegum and acacia. Diluents include lactose and sucrose. Sweetening agents include sucrose, syrups, glycerin and artificial sweetening agents such as saccharin. Wetting agents include propylene glycol monostearate, sorbitan monooleate, diethylene glycol monolaurate and polyoxyethylene lauryl ether. Organic acids include citric and tartaric acid. Sources of carbon dioxide include sodium bicarbonate and sodium carbonate. Coloring agents include any of the approved certified water soluble FD and C dyes, and mixtures thereof. Flavoring agents include natural flavors extracted from plants such fruits, and synthetic blends of compounds which produce a pleasant taste sensation.

For a solid dosage form, the solution or suspension, in for example propylene carbonate, vegetable oils or triglycerides, can be encapsulated in a gelatin capsule. Such solutions, and the preparation and encapsulation thereof, are disclosed in U.S. Patent Nos 4,328,245; 4,409,239; and 4,410,545. For a liquid dosage form, the solution, e.g., for example, in a polyethylene glycol, may be diluted with a sufficient quantity of a pharmaceutically acceptable liquid carrier, e.g., water, to be easily measured for administration.

Alternatively, liquid or semisolid oral formulations may be prepared by dissolving or dispersing the active compound or salt in vegetable oils, glycols, triglycerides, propylene glycol esters (*e.g.*, propylene carbonate) and other such carriers, and encapsulating these solutions or suspensions in hard or soft gelatin capsule shells. Other useful formulations include, but are not limited to, those containing a compound provided herein, a dialkylated mono- or poly-alkylene glycol, including, but not limited to, 1,2-dimethoxymethane, diglyme, triglyme, tetraglyme, polyethylene glycol-350-dimethyl ether, polyethylene glycol-550-dimethyl ether, polyethylene glycol-750-dimethyl ether wherein 350, 550 and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water-miscible solvents having one or more hydroxyl groups, including, but not limited to, propylene glycol and ethanol. Acetals include, but are not limited to, di(lower alkyl) acetals of lower alkyl aldehydes such as acetaldehyde diethyl acetal.

In all embodiments, tablets and capsules formulations may be coated as known by those of skill in the art in order to modify or sustain dissolution of the active ingredient. Thus, for example, they may be coated with a conventional enterically digestible coating, such as phenylsalicylate, waxes and cellulose acetate phthalate.

### Injectables, solutions and emulsions

Parenteral administration, generally characterized by injection, either subcutaneously, intramuscularly or intravenously is also contemplated herein. Injectables can be prepared in conventional forms, either as liquid solutions or suspensions, solid forms suitable for solution or suspension in liquid prior to injection, or as emulsions. Suitable excipients are, for example, water, saline, dextrose, glycerol or ethanol. In addition, if desired, the pharmaceutical compositions to be administered may also contain minor amounts of nontoxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins. Implantation of a slow release or sustained release system, such that a constant level of dosage is maintained is also contemplated herein. Briefly, a compound provided herein is dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The compound diffuses through the outer polymeric membrane in a release rate controlling step. The percentage of active compound contained in such parenteral compositions is highly dependent on the specific nature thereof, as well as the activity of the compound and the needs of the subject.

Parenteral administration of the compositions includes intravenous, subcutaneous and intramuscular administrations. Preparations for parenteral administration include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

If administered intravenously, suitable carriers include physiological saline or phosphate buffered saline (PBS), and solutions containing thickening and solubilizing agents, such as glucose, polyethylene glycol, and polypropylene glycol and mixtures thereof.

Pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles include Sodium Chloride Injection, Ringers Injection, Isotonic Dextrose Injection, Sterile Water Injection, Dextrose and Lactated Ringers Injection. Nonaqueous parenteral vehicles include fixed oils of vegetable origin, cottonseed oil, corn oil, sesame oil and peanut oil. Antimicrobial agents in bacteriostatic or fungistatic concentrations must be added to parenteral preparations packaged in multiple dose containers which include phenols or cresols, mercurials, benzyl alcohol, chlorobutanol, methyl and propyl phydroxybenzoic acid esters, thimerosal, benzalkonium chloride and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcelluose, hydroxypropyl methylcellulose and polyvinylpyrrolidone. Emulsifying agents include Polysorbate 80 (TWEEN® 80). A sequestering or chelating agent of metal ions includes EDTA. Pharmaceutical carriers also include ethyl alcohol, polyethylene glycol and propylene glycol for water miscible vehicles and sodium hydroxide, hydrochloric acid, citric acid or lactic acid for pH adjustment.

The concentration of the pharmaceutically active compound is adjusted so that an injection provides an effective amount to produce the desired pharmacological effect. The exact dose depends on the age, weight and condition of the patient or animal as is known in the art.

The unit dose parenteral preparations are packaged in an ampule, a vial or a syringe with a needle. All preparations for parenteral administration must be sterile, as is known and practiced in the art.

Illustratively, intravenous or intraarterial infusion of a sterile aqueous solution containing an active compound is an effective mode of administration. Another embodiment is a sterile aqueous or oily solution or suspension containing an active material injected as necessary to produce the desired pharmacological effect.

Injectables are designed for local and systemic administration. In certain embodiments, a therapeutically effective dosage is formulated to contain a concentration of at least about 0.1% w/w up to about 90% w/w or more, or more than 1% w/w of the active compound to the treated tissue(s). The active ingredient may be administered at once, or may be divided into a number of smaller doses to be administered at intervals of time. It is understood that the precise dosage and duration of treatment is a function of the tissue being treated and may be determined empirically using known testing protocols or by extrapolation from *in vivo* or *in vitro* test data. It is to be noted that concentrations and dosage values may also vary with the age of the individual treated. It is to be further understood that for any particular subject, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

The compound may be suspended in micronized or other suitable form or may be derivatized to produce a more soluble active product or to produce a prodrug. The form of the resulting mixture depends upon a number of factors, including the intended mode of administration and the solubility of the compound in the selected carrier or vehicle. The effective concentration is sufficient for ameliorating the symptoms of the condition and may be empirically determined.

### Lyophilized powders

Of interest herein are also lyophilized powders, which can be reconstituted for administration as solutions, emulsions and other mixtures. They may also be reconstituted and formulated as solids or gels.

The sterile, lyophilized powder is prepared by dissolving a compound provided herein, or a pharmaceutically acceptable derivative thereof, in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at about neutral pH. Subsequent sterile filtration of the solution followed by lyophilization under standard conditions known to those of skill in the art provides the desired formulation. Generally, the resulting solution will be apportioned into vials for lyophilization. Each vial will contain a single dosage (10-1000 mg or 100-500 mg) or multiple dosages of the compound. The lyophilized powder can be stored under appropriate conditions, such as at about 4 degrees Celsius to room temperature.

Reconstitution of this lyophilized powder with water for injection provides a formulation for use in parenteral administration. For reconstitution, about 1-50 mg, 5-35 mg or about 9-30 mg of lyophilized powder, is added per mL of sterile water or other suitable carrier. The precise amount depends upon the selected compound. Such amount can be empirically determined.

### Topical administration

Topical mixtures are prepared as described for the local and systemic administration. The resulting mixture may be a solution, suspension, emulsions or the like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The compounds or pharmaceutically acceptable derivatives thereof may be formulated as aerosols for topical application, such as by inhalation (see, *e.g.,* U.S. Patent Nos. 4,044,126, 4,414,209, and 4,364,923, which describe aerosols for delivery of a steroid useful for treatment of inflammatory diseases, particularly asthma). These formulations for administration to the respiratory tract can be in the form of an aerosol or solution for a nebulizer, or as a micro fine powder for insufflation, alone or in combination with an inert carrier such as lactose. In such a case, the particles of the formulation will have diameters of less than 50 microns or less than 10 microns.

The compounds may be formulated for local or topical application, such as for topical application to the skin and mucous membranes, such as in the eye, in the form of gels, creams, and lotions and for application to the eye or for intracisternal or intraspinal application. Topical administration is contemplated for transdermal delivery and also for administration to the eyes or mucosa, or for inhalation therapies. Nasal solutions of the active compound alone or in combination with other pharmaceutically acceptable excipients can also be administered.

These solutions, particularly those intended for ophthalmic use, may be formulated as 0.01% - 10% isotonic solutions, pH about 5-7, with appropriate salts.

### Compositions for other routes of administration

Other routes of administration, such as topical application, transdermal patches, and rectal administration are also contemplated herein. For example, pharmaceutical dosage forms for rectal administration are rectal suppositories, capsules and tablets for systemic effect. Rectal suppositories are used herein mean solid bodies for insertion into the rectum which melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glyceringelatin, carbowax (polyoxyethylene glycol) and appropriate mixtures of mono, di and triglycerides of fatty acids. Combinations of the various bases may be used. Agents to raise the melting point of suppositories include spermaceti and wax. Rectal suppositories may be prepared either by the compressed method or by molding. In certain embodiments, the weight of a rectal suppository is about 2 to 3 gm.

Tablets and capsules for rectal administration are manufactured using the same pharmaceutically acceptable substance and by the same methods as for formulations for oral administration.

### Sustained Release Compositions

Active ingredients such as the compounds provided herein can be administered by controlled release means or by delivery devices that are well known to those of ordinary skill in the art. Examples include, but are not limited to, those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358 and 6,699,500 each of which is incorporated herein by reference. Such dosage forms can be used to provide slow or controlled release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled release formulations known to those of ordinary skill in the art, including those described herein, can be readily selected for use with the active ingredients provided herein. Thus, the compositions provided encompasse single unit dosage forms suitable for oral administration such as, but not limited to, tablets, capsules, gelcaps, and caplets that are adapted for controlled release.

All controlled release pharmaceutical products have a common goal of improving drug therapy over that achieved by their non controlled counterparts. Ideally, the use of an optimally designed controlled release preparation in medical treatment is characterized by a minimum of drug substance being employed to cure or control the condition in a minimum amount of time. Advantages of controlled release formulations include extended activity of the drug, reduced dosage frequency, and increased subject compliance. In addition, controlled release formulations can be used to affect the time of onset of action or other characteristics, such as blood levels of the drug, and can thus affect the occurrence of side (*e.g*., adverse) effects.

Most controlled release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled release of an active ingredient can be stimulated by various conditions including, but not limited to, pH, temperature, enzymes, water, or other physiological conditions or compounds.

In certain embodiments, the drug may be administered using intravenous infusion, an implantable osmotic pump, a transdermal patch, liposomes, or other modes of administration. In one embodiment, a pump may be used (*see,* Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used. In yet another embodiment, a controlled release system can be placed in a subject at an appropriate site determined by a practitioner of skill, *i.e.,* thus requiring only a fraction of the systemic dose (*see, e.g.,* Goodson, Medical Applications of Controlled Release, vol. 2, pp. 115-138 (1984)). Other controlled release systems are discussed in the review by Langer (Science 249:1527-1533 (1990)). The active ingredient can be dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient in such parenteral compositions is highly dependent on the specific nature thereof, as well as the needs of the subject.

### Targeted Formulations

The compounds provided herein, or pharmaceutically acceptable derivatives thereof, may also be formulated to be targeted to a particular tissue, receptor, or other area of the body of the subject to be treated. Many such targeting methods are well known to those of skill in the art. All such targeting methods are contemplated herein for use in the instant compositions. For non-limiting examples of targeting methods, see, *e.g.,* U.S. Patent Nos. 6,316,652, 6,274,552, 6,271,359, 6,253,872, 6,139,865, 6,131,570, 6,120,751, 6,071,495, 6,060,082, 6,048,736, 6,039,975, 6,004,534, 5,985,307, 5,972,366, 5,900,252, 5,840,674, 5,759,542 and 5,709,874.

In one embodiment, liposomal suspensions, including tissue-targeted liposomes, such as tumor-targeted liposomes, may also be suitable as pharmaceutically acceptable carriers. These may be prepared according to methods known to those skilled in the art. For example, liposome formulations may be prepared as described in U.S. Patent No. 4,522,811. Briefly, liposomes such as multilamellar vesicles (MLV's) may be formed by drying down egg phosphatidyl choline and brain phosphatidyl serine (7:3 molar ratio) on the inside of a flask. A solution of a compound provided herein in phosphate buffered saline lacking divalent cations (PBS) is added and the flask shaken until the lipid film is dispersed. The resulting vesicles are washed to remove unencapsulated compound, pelleted by centrifugation, and then resuspended in PBS.

### Dosage and Unit Dosage Forms

In human therapeutics, the doctor will determine the posology which he considers most appropriate according to a preventive or curative treatment and according to the age, weight, stage of the disease and other factors specific to the subject to be treated. Generally, doses are from about 1 to about 1000 mg per day for an adult, or from about 5 to about 250 mg per day or from about 10 to 50 mg per day for an adult. In certain embodiments, doses are from about 5 to about 400 mg per day or 25 to 200 mg per day per adult. Dose rates of from about 50 to about 500 mg per day are also contemplated.

In certain embodiments, the amount of the compound or composition which will be effective in the prevention or treatment of the liver disease or one or more symptoms thereof will vary with the nature and severity of the disease or condition, and the route by which the active ingredient is administered. The frequency and dosage will also vary according to factors specific for each subject depending on the specific therapy (*e.g*., therapeutic or prophylactic agents) administered, the severity of the disorder, disease, or condition, the route of administration, as well as age, body, weight, response, and the past medical history of the subject. Effective doses may be extrapolated from dose-response curves derived from *in vitro* or animal model test systems.

Exemplary doses of a composition include milligram or microgram amounts of the and caspase inhibitor per kilogram of subject or sample weight (*e.g*., about 10 micrograms per kilogram to about 50 milligrams per kilogram, about 100 micrograms per kilogram to about 25 milligrams per kilogram, or about 100 microgram per kilogram to about 10 milligrams per kilogram). In certain embodiments, the dosage administered to a subject is between 0.20 mg/kg and 2.00 mg/kg, or between 0.30 mg/kg and 1.50 mg/kg of the subject's body weight.

In certain embodiments, the recommended daily dose range of the and caspase inhibitor described herein for the conditions described herein lies within the range of from about 0.1 mg to about 1000 mg of each of the and caspase inhibitor per day, given as a single once-a-day dose or as divided doses throughout a day. In one embodiment, the daily dose is administered twice daily in equally divided doses. Specifically, a daily dose range should be from about 10 mg to about 200 mg per day, more specifically, between about 10 mg and about 150 mg per day, or even more specifically between about 25 and about 100 mg per day. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with subject response.

Different therapeutically effective amounts may be applicable for different diseases and conditions, as will be readily known by those of ordinary skill in the art. Similarly, amounts sufficient to prevent, manage, treat or ameliorate such disorders, but insufficient to cause, or sufficient to reduce, adverse effects associated with the compound described herein are also encompassed by the above described dosage amounts and dose frequency schedules. Further, when a subject is administered multiple dosages of a compound described herein, not all of the dosages need be the same. For example, the dosage administered to the subject may be increased to improve the prophylactic or therapeutic effect of the compound or it may be decreased to reduce one or more side effects that a particular subject is experiencing.

In one embodiment, the dosage of compounds described herein administered to prevent, treat, manage, or ameliorate a disorder, or one or more symptoms thereof in a subject is 0.1 mg/kg, 1 mg/kg, 2 mg/kg, 3 mg/kg, 4 mg/kg, 5 mg/kg, 6 mg/kg, 10 mg/kg, or 15 mg/kg or more of a subject's body weight. In another embodiment, the dosage of the compounds provided herein administered to prevent, treat, manage, or ameliorate a disorder, or one or more symptoms thereof in a subject is a unit dose of 0.1 mg to 200 mg, 0.1 mg to 100 mg, 0.1 mg to 50 mg, 0.1 mg to 25 mg, 0.1 mg to 20 mg, 0.1 mg to 15 mg, 0.1 mg to 10 mg, 0.1 mg to 7.5 mg, 0.1 mg to 5 mg, 0.1 to 2.5 mg, 0.25 mg to 20 mg, 0.25 to 15 mg, 0.25 to 12 mg, 0.25 to 10 mg, 0.25 mg to 7.5 mg, 0.25 mg to 5 mg, 0.5 mg to 2.5 mg, 1 mg to 20 mg, 1 mg to 15 mg, 1 mg to 12 mg, 1 mg to 10 mg, 1 mg to 7.5 mg, 1 mg to 5 mg, or 1 mg to 2.5 mg.

In certain embodiments, treatment or prevention can be initiated with one or more loading doses of a caspase inhibitor provided herein followed by one or more maintenance doses. In such embodiments, the loading dose can be, for instance, about 60 to about 400 mg per day, or about 100 to about 200 mg per day for one day to five weeks. The loading dose can be followed by one or more maintenance doses. Each maintenance does can be, independently, about from about 10 mg to about 200 mg per day, more specifically, between about 25 mg and about 150 mg per day, or even more specifically between about 25 mg and about 80 mg per day or between about 25 mg and about 50 mg per day. Maintenance doses can be administered daily and can be administered as single doses, or as divided doses.

In certain embodiments, a dose of a caspase inhibitor provided herein can be administered to achieve a steady-state concentration of the active ingredient in blood or serum of the subject. The steady-state concentration can be determined by measurement according to techniques available to those of skill or can be based on the physical characteristics of the subject such as height, weight and age. In certain embodiments, a sufficient amount of a compound provided herein is administered to achieve a steady-state concentration in blood or serum of the subject of from about 300 to about 4000 ng/mL, from about 400 to about 1600 ng/mL, or from about 600 to about 1200 ng/mL. Loading doses can be administered to achieve steady-state blood or serum concentrations of about 1200 to about 8000 ng/mL, or about 2000 to about 4000 ng/mL for one to five days. Maintenance doses can be administered to achieve a steady-state concentration in blood or serum of the subject of from about 300 to about 4000 ng/mL, from about 400 to about 1600 ng/mL, or from about 600 to about 1200 ng/mL.

In certain embodiments, administration of the same compound may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same prophylactic or therapeutic agent may be repeated and the administration may be separated by at least at least 1 day, 2 days, 3 days, 5 days, 10 days, 15 days, 30 days, 45 days, 2 months, 75 days, 3 months, or 6 months.

In certain aspects, provided herein are unit dosages comprising a compound, or a pharmaceutically acceptable derivative thereof, in a form suitable for administration. Such forms are described in detail above. In certain embodiments, the unit dosage comprises 1 to 1000 mg, 5 to 250 mg or 10 to 50 mg active ingredient. In particular embodiments, the unit dosages comprise about 1, 5, 10, 25, 50, 100, 125, 250, 500 or 1000 mg active ingredient. Such unit dosages can be prepared according to techniques familiar to those of skill in the art.

### Articles of manufacture

The compounds or pharmaceutically acceptable derivatives can be packaged as articles of manufacture containing packaging material, a compound or pharmaceutically acceptable derivative thereof provided herein, which is used for treatment, prevention or amelioration of one or more symptoms associated with liver disease, and a label that indicates that the compound or pharmaceutically acceptable derivative thereof is used for treatment, prevention or amelioration of one or more symptoms of liver diseases.

The articles of manufacture provided herein contain packaging materials. Packaging materials for use in packaging pharmaceutical products are well known to those of skill in the art. See, e.g., U.S. Patent Nos. 5,323,907, 5,052,558 and 5,033,252. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of formulations of the compounds and compositions provided herein are contemplated.

### Kit

Further provided are kits for use in methods of treatment of a liver disease. The kits can include a caspase inhibitor or composition thereof, and instructions providing information to a health care provider regarding usage for treating or preventing the liver disease. Instructions may be provided in printed form or in the form of an electronic medium such as a CD, or DVD, or in the form of a website address where such instructions may be obtained. A unit dose of an or composition thereof, or a caspase inhibitor or composition thereof, can include a dosage such that when administered to a subject, a therapeutically or prophylactically effective plasma level of the compound or composition can be maintained in the subject for at least 1 day. In some embodiments, the compounds or composition can be included as sterile aqueous pharmaceutical compositions or dry powder (*e.g*., lyophilized) compositions.

### 5.6. Evaluation Of The Activity Of The Compounds

The biological activity of the compounds can be demonstrated by methods known to one of skill in the art. For example, as described by Abraldes, J. el al. Animal models of portal hypertension. World J. Gastroenterology 2006;41:6577-6584. Geerts, A. et al. Comparison of three research models of portal hypertension in mice: macroscopic, histological and portal pressure evaluation. Int. J. Exp. Path. 2008;89:251-263. and Guerra, R. et al. A novel chronic cirrhosis TAA-induced model in rats. Braz. J. Vet. Pathol. 2010;3:9-16 which are incorporated herein by reference in their entirety.

Multiple outcome measures in circulation and tissue can used for evaluation. One of these is the measurement of levels of the liver enzyme ALT in the blood. Elevated ALT levels are routinely observed in the blood of patients suffering from a variety of liver diseases. ALT measurement is a very common and relevant clinical laboratory test for the extent of liver disease in patients. A second measure involves gross and histological evaluation of the extent liver disease. Histology is often done in patients with advanced liver disease to determine the extent of disease. The extent of liver disease can be graded by examining liver samples prepared and evaluated microscopically by trained observers. In certain embodiment, the liver injury can be sufficiently severe as to cause mortality. In certain embodiment, compounds described herein protect against induced liver injury as determined by these parameters.

### 5.7. Combination Therapy

In certain embodiments, caspase inhibitors provided herein are administered in combination with one or more agents known to treat portal hypertension and / or cirrhosis. In certain embodiments, dosages lower than those which have been or are currently being used to treat portal hypertension and / or cirrhosis combination therapies provided herein. For those agents that are approved for clinical use, recommended dosages are described in, for example, Hardman et al., eds., 1996, Goodman & Gilman's The Pharmacological Basis Of Basis Of Therapeutics 9th Ed, Mc-Graw-Hill, New York; Physician's Desk Reference (PDR) 57th Ed., 2003, Medical Economics Co., Inc., Montvale, NJ, which are incorporated herein by reference in their entireties. The dosages given will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions.

In various embodiments, the compounds provided herein are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In certain embodiments, two or more therapies are administered within the same patient visit.

In certain embodiments, the compounds provided herein and optionally an additional agent are administered to a patient, for example, a mammal, such as a human, in a sequence and within a time interval such that the compounds provided herein can act together with the other agent to provide an increased benefit than if they were administered otherwise. For example, the compounds can be administered at the same time or sequentially in any order at different points in time; however, if not administered at the same time, they should be administered sufficiently close in time so as to provide the desired therapeutic or prophylactic effect. In one embodiment, the compounds provided herein and optionally an additional agent exert their effect at times which overlap. Each compound can be administered separately, in any appropriate form and by any suitable route. In other embodiments, the compounds provided herein are administered before, concurrently or after administration of the first compound.

The caspase inhibitor compounds provided herein and optionally one or more additional agents can act additively or synergistically. In one embodiment, the caspase inhibitor compounds provided herein can act additively or synergistically with another agent. In one embodiment, the compounds provided herein are administered concurrently, optionally with another agent, with in the same pharmaceutical composition. In another embodiment, the compounds provided herein are administered concurrently, optionally with another agent, in separate pharmaceutical compositions. In still another embodiment, the compounds provided herein are administered with another agent, prior to or subsequent to administration of the third agent. Also contemplated are administration of the compounds provided herein by the same or different routes of administration, *e.g.*, oral and parenteral.

In certain embodiments, the additional agents administered in combination with caspase inhibitors according to the methods provided herein can include-products currently used in the treatment of patients with portal hypertension: Propranolol, Nadolol, Carvedilol and analogs or derivatives thereof as understood by those of skill in the art.

In certain embodiments, the additional agents administered in combination with caspase inhibitors according to the methods provided herein can include, but are not limited to, any compounds currently in preclinical or clinical development for treatment of portal hypertension and / or cirrhosis: Simtuzumab (GS-6624) by Gilead, Sorafenib by Bayer and Onyx, Serelaxin (RLX030) by Norvartis, Timolol, NCX-1000, Terlipressin, NGM282, LUM001, and analogs or derivatives thereof as understood by those of skill in the art.

The compounds provided herein can also be administered in combination with antibiotics, antiviral compounds, antifungal agents or other pharmaceutical agents administered for the treatment of infections: rifaximin, neomycin, cefotaximine, ciprofloxacin, norfloxacin, lactulose, and analogs or derivatives thereof as understood by those of skill in the art.

It is understood that the foregoing detailed description and accompanying examples are merely illustrative, and are not to be taken as limitations upon the scope of the subject matter. Various changes and modifications to the disclosed embodiments will be apparent to those skilled in the art. Such changes and modifications, including without limitation those relating to the chemical structures, substituents, derivatives, intermediates, syntheses, formulations and/or methods of use herein, may be made without departing from the spirit and scope thereof. U.S. patents and publications referenced herein are incorporated by reference.

### 6. EXAMPLES

### EXAMPLE 1.

Emricasan was prepared as described in Linton S. et al. J. Med Chem. 2005;48:6779.

### Part A: Methyl 2-[N-(2-tert-Butylphenylamino)]-2-oxoacetate

A solution of 2-tert-butylaniline (57 mL, 54.5 g, 366 mmol), triethylamine (56 mL, 402 mmol), and methylene chloride (370 mL) was cooled to 0oc (ice bath) and stirred under nitrogen. An addition funnel was charged with methyl 2-chloro-2-oxoacetate (50 g, 408 mmol) which was then added dropwise over 20 minutes to the stirred solution causing a significant exotherm. After addition complete, the resulting suspension was stirred for 1 hr. The suspension was then concentrated under vacuum, which is then taken up in ethyl acetate and partitioned with water. The aqueous layer was washed twice with ethyl acetate, and the combined organic layers were then extracted with 5% aqueous potassium bisulfate, followed by saturated sodium chloride, and then dried over magnesium sulfate and concentrated under reduced pressure. The resulting oil was then dried overnight, then recrystallized from 3:1 hexanes/toluene (two crops) to give the title compound as a white crystalline solid (60.43 g, 70 %)

### Part B: N-(2-tert-Butylphenylamino)oxamic acid

To a solution of methyl 2-[N-(2-tert-Butylphenylamino)]-2-oxoacetate (59.8 g, 254 mmol) in 1,4-dioxane (600 mL) was slowly added IN lithium hydroxide (300 mL, 300 mmol). The solution was stirred for 1 hour. The solution was then acidified dropwise with concentrated HCl (12M, 25.0 mL, 300 mmol), and the resulting solution was extracted with ethyl acetate (3 times) and the combined organic extracts were then washed with saturated sodium chloride, dried over magnesium sulfate, and concentrated under vacuum and recrystallized from ethyl acetate/hexanes to yield the title compound (32.55 g, 58%)

### Part C: [(N-Benzyloxycarbonyl)Alaninyl]Aspartic Acid, [beta]-tert-Butyl Ester

To a suspension of aspartic acid b-tert-butyl ester (3.784 g, 20 mmol) in dimethylformamide (150 mL) at room temperture under nitrogen was added bis(trimethylsilyl)-trifluoroacetamide (10.6 mL, 40 mmol). After stirring at room temperature for 30 min, the resulting clear solution was treated with (N-benzyloxycarbonyl) alanine N-hydroxysuccinimide ester (6.406 g, 20 mmol). After stirring at room temperature for 18 hours, the mixture was treated with water (20 mL), stirred for 15 minutes and then partitioned between ethyl acetate & water. The organic phase was washed with water, 5% potassium bisulfate and saturated sodium chloride solutions, dried over anhydrous sodium sulfate, and evaporated to dryness. The residue was then taken up in ethyl ether and extracted with saturated sodium bicarbonate. The aqueous extract was acidified (pH 2.0) with concentrated HCl and extracted with ethyl acetate. The ethyl acetate extract was washed with saturated sodium chloride solution, dried over anhydrous sodium Part D: (3S,4RS)-3-[(N-Benzyloxycarbonyl)Alaninyl]Amino-5-Bromo-4-Oxopentanoic Acid tert-Butyl Ester

### Part D: (3S,4RS)-3-[(N-Benzyloxycarbonyl)Alaninyl]Amino-5-Bromo-4-Oxopentanoic Acid tert-Butyl Ester

A solution of [(N-benzyloxycarbonyl)alaninyl]aspartic acid, beta-tert-butylester (5.0 g, 12.7 mmol) and N-methylmorpholine (2.05 g, 2.23 mL, 20.3 mmol) in tetrahydrofuran (65 mL) at -10°C. (NaCl/ice bath) under nitrogen was treated dropwise with isobutylchloroformate (2.6 g, 2.47 mL, 19.04 mmol). After stirring at -10°C for 20 minutes, the mixturewas filtered (sintered glass) into a pre-cooled receiver (ice bath) washing the filter cake with additional tetrahydrofuran (approx. 48 mL). The combined filtrate was treated with excess diazomethane/ethyl ether solution (prepared from 4.67 g, 31.73 mmol of 1-methyl-3-nitro-1-nitrosoguanidine, 34 mL 40% KOH/ 85 ml ethyl ether) at 0°C (ice bath) under nitrogen. After stirring at 0°C for 15 minutes and at room temperature for 30 minutes, the reaction mixture was again cooled to 0°C and treated with 48% HBr in acetic acid (34 mL, 204 mmol)/acetic acid (34 mL). After stirring at 0°C for 15 minutes and at room temperature for 30 minutes, the mixture was partitioned between ethyl acetate & water. The organic phase was washed successively with water, saturated sodium bicarbonate, and saturated sodium chloride; dried over anhydrous sodium sulfate and evaporated to dryness and purified by flash chromatography on silica gel eluting with ethyl acetate-hexane (1:2) to give the title compound as a white foam (3.12 g, 52%). Part E: (3S,4RS)-3-[(N-Benzyloxycarbonyl)Alaninyl]Amino-5-(2',3',5',6'-Tetrafluorophenoxy)-4-Oxopentanoic Acid tert-Butyl Ester

To a solution of (3S)-3-[(N-benzyloxycarbonyl)alaninyl]amino-5-bromo-4-oxopentanoic acid tert-butyl ester (0.167 g, 0.355 mmol) and 2,3,5,6-tetrafluorophenol (0.071 g, 0.426 mmol) in N,N-dimethylformamide (2 mL) at room temperature under nitrogen was added potassium fluoride (0.082 g, 1.42 mmol). After stirring at room temperature for 4 hrs, the mixture was diluted with ethyl acetate, washed with saturated sodium bicarbonate and saturated sodium chloride solutions, dried over anhydrous sodium sulfate and evaporated to dryness. The crude material (0.144 g) was taken on to the next step without purification.

### Part F: (3S,4RS)-3-[(N-Benzyloxycarbonyl)Alaninyl]Amino-5-(2',3',5',6'-Tetrafluorophenoxy)-4-Hydroxypentanoic Acid tert-Butyl Ester

To a solution of crude (3S)-3-[(N-benzyloxycarbonyl)alaninyl]amino-5-(2',3',5',6'- tetrafluorophenoxy)-4-oxopentanoic acid tert-butyl ester (0.144 g, 0.26 mmol) in 1:1 methanol/tetrahydrofuran (4mL) at 0°C under nitrogen was added sodium borohydride (0.040 g, 1.04 mmol). After stirring at 0°C for 1 hour, the mixture was concentrated and the residue partitioned between ethyl acetate-half saturated ammonium chloride solution (50% saturated ammonium chloride/ 50% water). The organic phase was washed with saturated sodium bicarbonate and saturated sodium chloride solutions, dried over anhydrous sodium sulfate and evaporated to dryness. The residue was purified by flash chromatography on silica gel eluting with ethyl acetate-hexanes (1:2) to give the title compound (0.142 g, 78%) as a white foam. Part G: (3S,4RS)-3-(Alaninyl)Amino-5-(2',3',5',6'-Tetrafluorophenoxy)-4-Hydroxypentanoic Acid tert-Butyl Ester

To a solution of (3S, 4RS)-3-[(N-benzyloxycarbonyl)valinyl]amino-5-(2',3',5',6'-tetrafluorophenoxy)-4-hydroxypentanoic acid tert-butyl ester (0.112 g, 0.201 mmol) in methanol (10 mL) was added 10% Pd-C (0.017 g) and resulting mixture stirred under a hydrogen atmosphere (1 atmosphere, balloon) for 2 hrs. The mixture was filtered through Celite, washing the filter cake with methanol. The combined filtrates evaporated to dryness to yield the crude title compound as a colorless, viscous oil (0.066 g, 70%) which was taken on to the next step without purification.

### Part H: (3S,4RS)-3-[N-(N'-(2-tert-Butylphenyl)Oxamyl) Alaninyl]Amino-5-(2',3',5',6'-Tetrafluorophenoxy)-4-Hydroxypentanoic Acid tert-Butyl Ester

To a solution of N-(2-tert-butylphenyl)oxamic acid (0.041 g, 0.19 mmol) in methylene chloride (6.0 mL) at 0°C under nitrogen was added hydroxybenzotriazole hydrate (0.030 g, 0.261 mmol) followed by addition of 1-ethyl-3-(3',3'-dimethyl-1'-aminopropyl)-carbodiimide hydrochloride (EDCI) (0.050 g, 0.26 mmol). After stirring at 0°C for 10 min, the mixture was treated with (3S,4RS)-3-(alaninyl)amino-5-(2',3',5',6'-tetrafluorophenoxy)-4-hydroxypentanoic acid tert-butyl ester (0.079 g, 0.19 mmol) and N-methylmorpholine (NMM) (22 mL, 0.20 mmol). After stirring at room temperature for 16 hrs, the mixture was partitioned between ethyl acetate & water. The organic phase was washed with water, 5% potassium bisulfate, saturated sodium bicarbonate and saturated sodium chloride solutions, dried over anhydrous sodium sulfate and evaporated to give the crude title compound (0.090 g, 77%) as a viscous oil.

### Part I: (3S)-3-[N-(N'-(2-tert-Butylphenyl) Oxamyl)Alaninyl]Amino-5-(2',3',5',6'-Tetrafluorophenoxy)-4-Oxopentanoic Acid tert-Butyl Ester

To a solution of (3S,4RS)-3-[N-(N'-(2-tert-butylphenyl)oxamyl)alaninyl]amino-5-(2',3',5',6'- tetrafluorophenoxy)-4-hydroxypentanoic acid tert-butyl ester (0.0.092 g, ca 0.15 mmol) in methylene chloride (6.5 mL) at room temperature under nitrogen was added iodobenzene diacetate (0.188 g, 0.58 mmol) followed by a catalytic amount of 2,2,6,6-tetramethyl-1-piperidinyloxy free radical (TEMPO, 0.0046 g, 0.03 mmol). After stirring at room temperature for 16 hrs, the mixture was partitioned between ethyl acetate & water. The organic phase was washed with saturated sodium bicarbonate and saturated sodium chloride solutions, dried over anhydrous sodium sulfate and evaporated to dryness. The residue (0.096 g) was purified by preparative layer chromatography on silica gel eluting with ethyl acetate-hexane (3:7) to give the title compound (0.071 g, 77%) as a colorless glass.

### Part J: (3S)-3-[N-(N'-(2-tert-Butylphenyl) Oxamyl)Alaninyl]Amino-5-(2',3',5',6'-Tetrafluorophenoxy)-4-Oxopentanoic Acid

To a solution of (3S)-3-[N-(N'-(2-tert-butylphenyl)oxamyl)alaninyl]amino-5-(2',3',5',6'- tetrafluorophenoxy)-4-oxopentanoic acid, tert-butyl ester (0.071 g, 0.11 mmol) and anisole (0.05 mL) in methylene chloride (2.5 mL) at room temperature under nitrogen was added trifluoroacetic acid (1.5 mL). The resulting clear solution was stirred at room temperature for 1 hr, evaporated to dryness and chased with toluene-methylene chloride (1:1). The residue (0.061g) was purified by preparative layer chromatography on silica gel eluting with methanol-methylene chloride (1:9) to give the title compound (0.044 g, 69%) as a colorless glass.

The embodiments described above are intended to be merely exemplary, and those skilled in the art will recognize, or will be able to ascertain using no more than routine experimentation, numerous equivalents of specific compounds, materials, and procedures. All such equivalents are considered to be within the scope of the claimed subject matter and are encompassed by the appended claims.

The application discloses inter alia the following items:
1. A method of treating a complication of a chronic liver disease, comprising administering, to a subject in need thereof, a therapeutically effective amount of a caspase inhibitor, whereby the complication of the chronic liver disease is mitigated.
2. The method of item 1, wherein the complication is portal hypertension or cirrhosis.
3. A method of treating a complication of a chronic liver disease, comprising administering, to a subject in need thereof, a therapeutically effective amount of a caspase inhibitor, said amount being effective to treat the subject by mitigating a consequence of the chronic liver disease.
4. The method of item 3, wherein the consequence is portal hypertension or cirrhosis.
5. The method of any of items 1-4, wherein the caspase inhibitor is selected from: 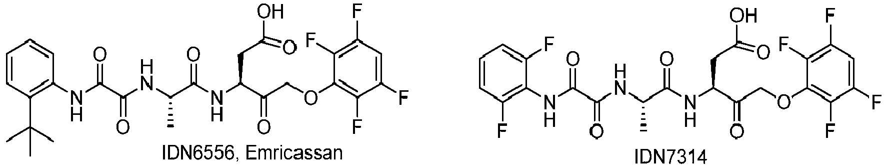 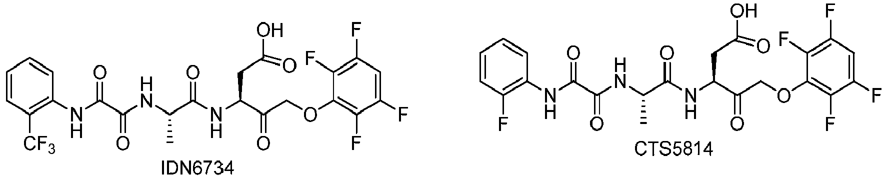 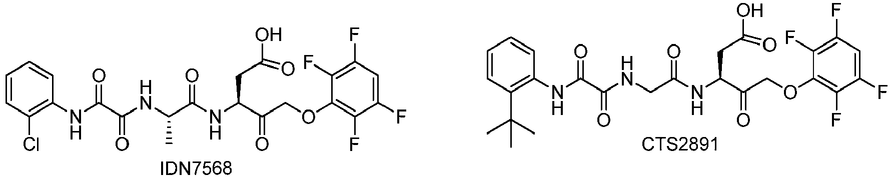 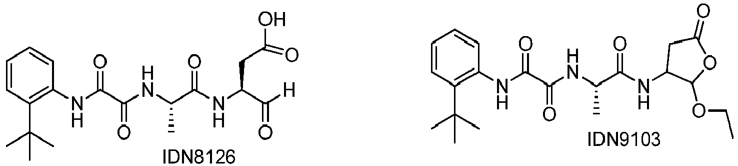 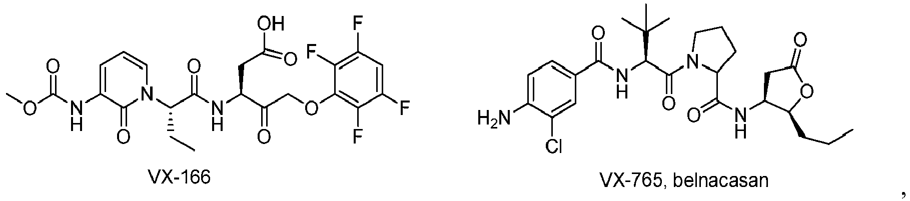 or a pharmaceutically acceptable derivative thereof.
6. A method according to any of items 1-5, wherein the caspase inhibitor is selected from: 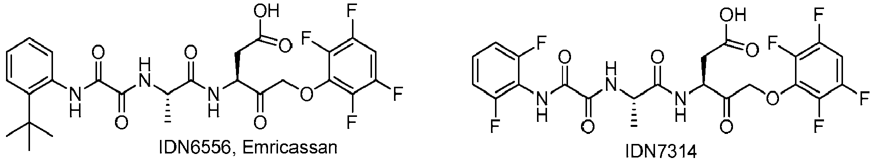 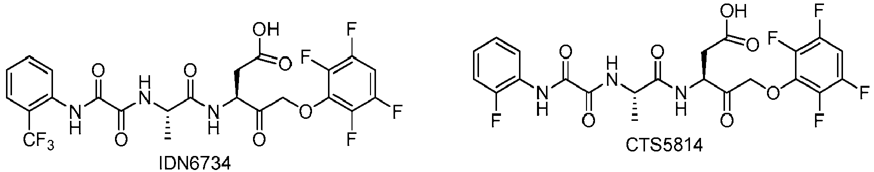 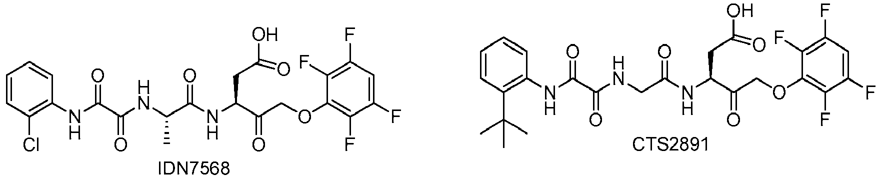 or a pharmaceutically acceptable derivative thereof.
7. A method according to any of items 1-5, wherein the caspase inhibitor is selected from: 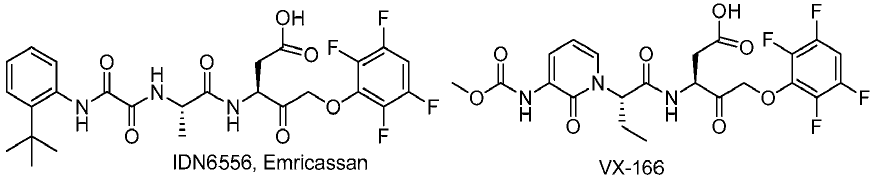 or a pharmaceutically acceptable derivative thereof.
8. The method according to item 7, wherein the caspase inhibitor is: 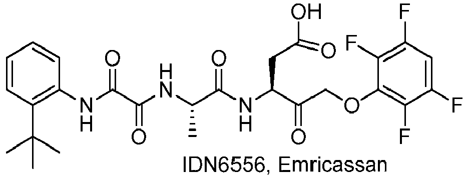 or a pharmaceutically acceptable derivative thereof.
9. A method according to item 7, wherein the caspase inhibitor is: 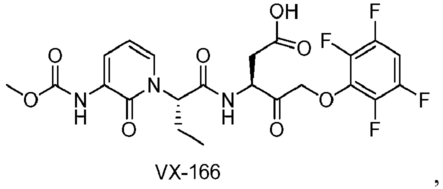 or a pharmaceutically acceptable derivative thereof.
10. A method according to any of items 1-5, wherein the caspase inhibitor is selected from: 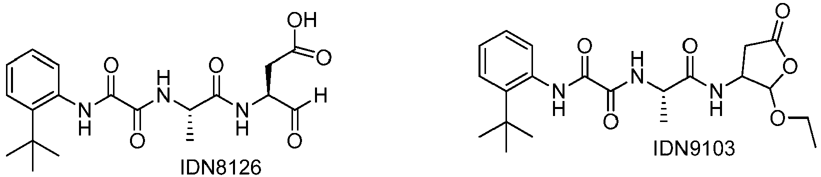 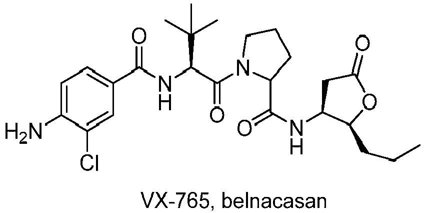 or a pharmaceutically acceptable derivative thereof.
11. A method according to item 10, wherein the caspase inhibitor is: 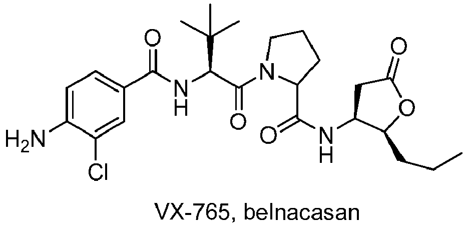 or a pharmaceutically acceptable derivative thereof.
12. A method according to any of items 1-5 wherein the caspase inhibitor is: 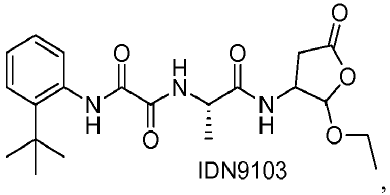 or a pharmaceutically acceptable derivative thereof.
13. The method of any of items 1-12, wherein the complication or consequence of a chronic liver disease is selected from among portal hypertension, esophageal varices, ascites, hepatic encephalopathy, variceal hemorrhage and hepatocellular carcinoma.
14. The method of item 13, wherein the complication of chronic liver disease is portal hypertension.
15. The method of item 13, wherein the complication of chronic liver disease is cirrhosis.
16. The method of any of items 1-15, wherein the patient has been pre-treated with one or more other medications for the complication of chronic liver disease.
17. The method of item 16, wherein the one or more other medications are selected from among Propranolol, Nadolol, Carvedilol, Simtuzumab (GS-6624), Sorafenib, Serelaxin (RLX030), Timolol, NCX-1000, Terlipressin, NGM282 and LUM001, and analogs or derivatives thereof.
18. The method of any of items 1-17, wherein the patient has failed therapy for the complication of chronic liver disease.
19. The method of any of items 1-17, wherein the patient has failed therapy for portal hypertension.
20. The method of any of items 1-17, wherein the patient has failed therapy for liver cirrhosis.
21. A method of treating portal hypertension, comprising administering, to a subject in need thereof, a therapeutically effective amount of a caspase inhibitor, the amount being effective to treat the subject by mitigating portal hypertension in the subject.
22. A method of treating portal hypertension, comprising administering, to a subject in need thereof, a therapeutically effective amount of a caspase inhibitor, said amount being effective to treat the subject by lowering elevated levels of liver enzyme in the subject.
23. The method of item 22, wherein the levels are of total liver enzyme, alanine aminotransferase or aspartate aminotransferase.
24. The method of item 22 or item 23, wherein the elevated level of liver enzyme is lowered by about 100% to about 1%.
25. The method of item 24, wherein the elevated level of liver enzyme is lowered by at least 99%, at least 90%, at least 80%, at least 70%, at least 60%, at least 50%, at least 40%, at least 30%, at least 20%, at least 10%, at least 5%, at least 2% or at least 1%.
26. A method for inhibiting a signaling cascade of TNF-α comprising administering, to a subject, a therapeutically effective amount of a caspase inhibitor, said amount being effective to inhibit the signaling cascade of TNF-α in the subject.
27. A method for inhibiting a signaling cascade of α-Fas comprising administering, to a subject in need thereof, a therapeutically effective amount of a caspase inhibitor or a pharmaceutically acceptable derivative thereof, said amount being effective to inhibit the signaling cascade of α -Fas in the subject.
28. The method of any of items 21-27, wherein the caspase inhibitor is selected from: 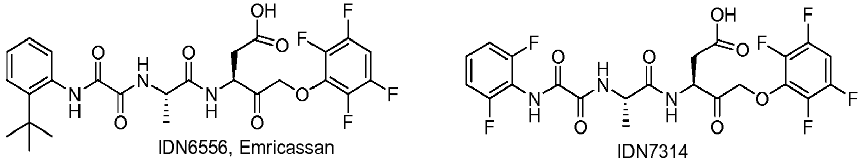 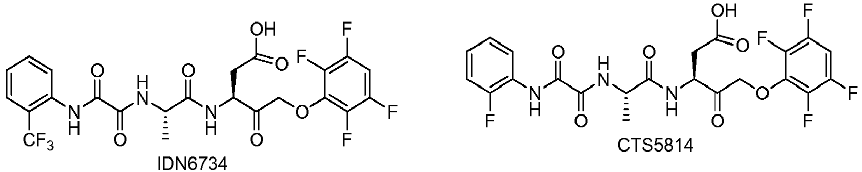 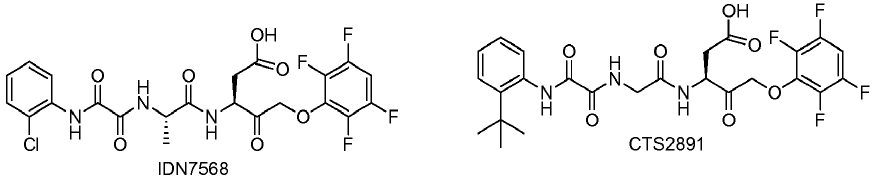 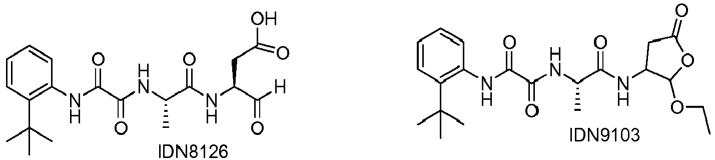 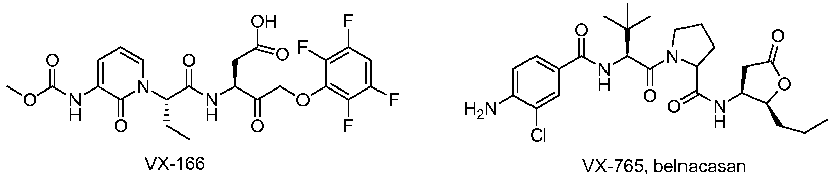 or a pharmaceutically acceptable derivative thereof.
29. A method according to any of items 21-28, wherein the caspase inhibitor is selected from: 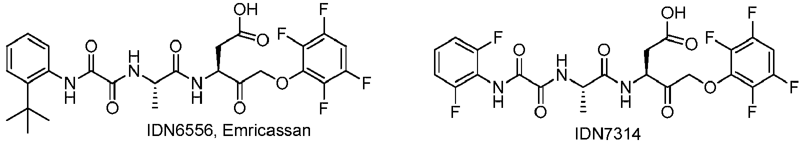 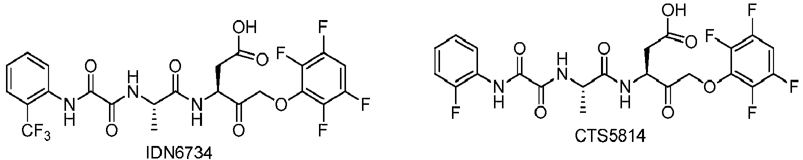 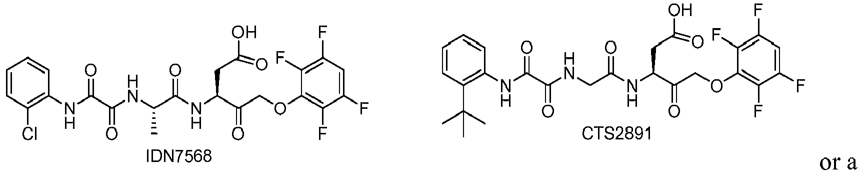 pharmaceutically acceptable derivative thereof.
30. A method according to any of items 21-28, wherein the caspase inhibitor is selected from: 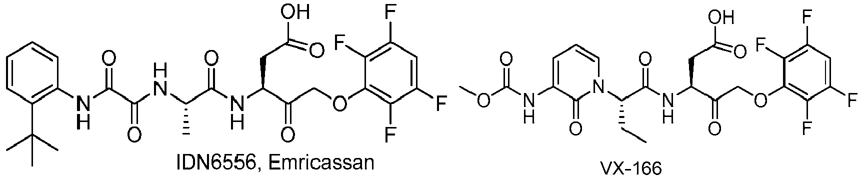 or a pharmaceutically acceptable derivative thereof.
31. The method of item 30, wherein the caspase inhibitor is: 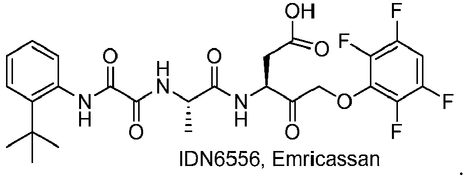
32. A method according to item 30, wherein the caspase inhibitor is: 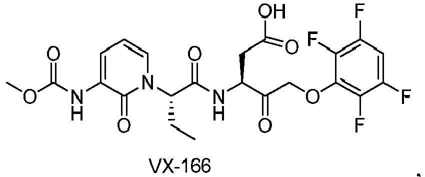 or a pharmaceutically acceptable derivative thereof.
33. A method according to any of items 21-28, wherein the caspase inhibitor is selected from: 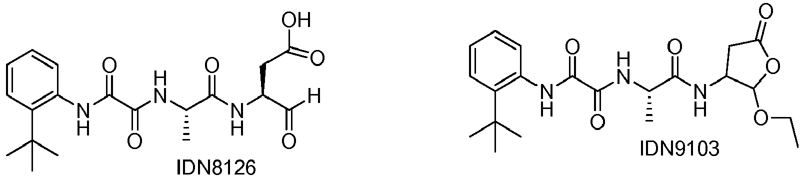 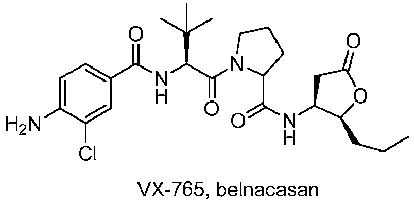 or a pharmaceutically acceptable derivative thereof.
34. A method according to item 33, wherein the caspase inhibitor is: 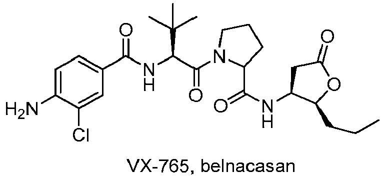 or a pharmaceutically acceptable derivative thereof.
35. A method according to item 33, wherein the caspase inhibitor is: 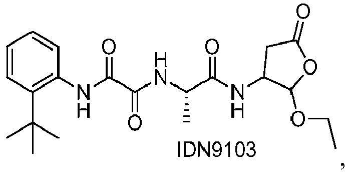 or a pharmaceutically acceptable derivative thereof.
36. A pharmaceutical composition, comprising a caspase inhibitor and a pharmaceutically acceptable excipient, wherein the caspase inhibitor is in an amount that is therapeutically effective in the treatment of portal hypertension or cirrhosis.
37. The composition of item 36 that is formulated for oral administration.
38. The composition of item 36 that is formulated for nasogastric administration.
39. The composition of item 36, wherein the caspase inhibitor is in amount of about 1 mg to about 100 mg.
40. The composition of item 39, wherein the caspase inhibitor is in an amount of about 25 mg to about 50 mg.
41. The composition of any of items 36-40, wherein the caspase inhibitor is administered at least once daily.
42. The composition according to any of items 36-41, wherein the caspase inhibitor is selected from: 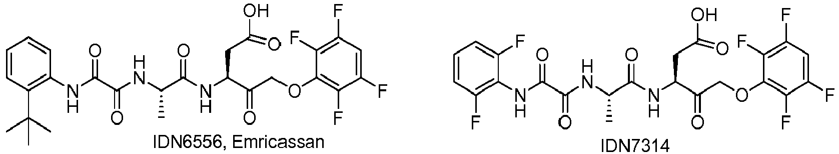 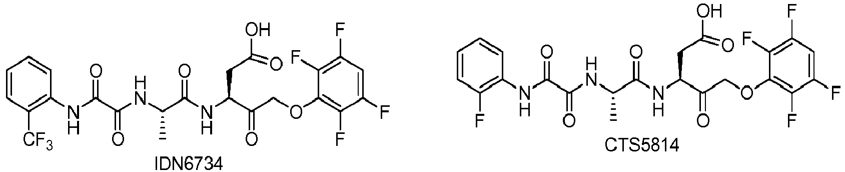 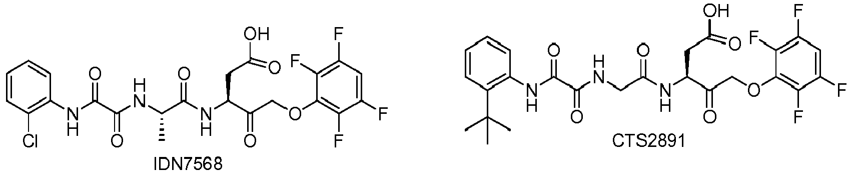 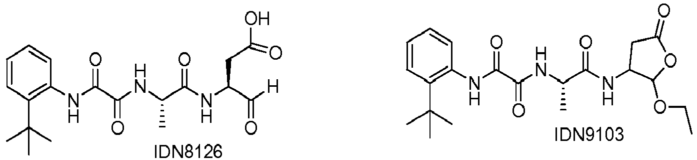 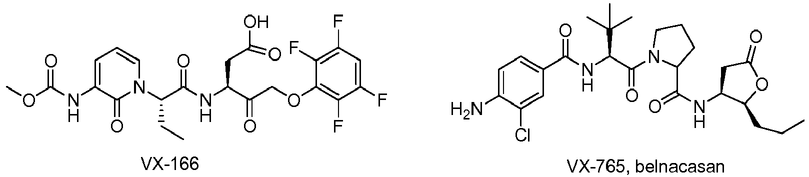 or a pharmaceutically acceptable derivative thereof.
43. The composition according to any of items 36-41, wherein the caspase inhibitor is selected from: 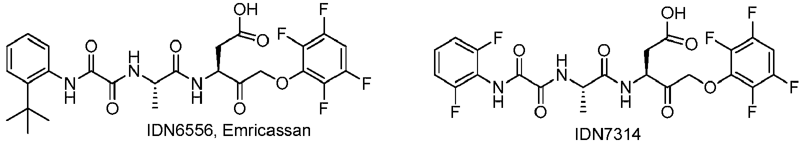 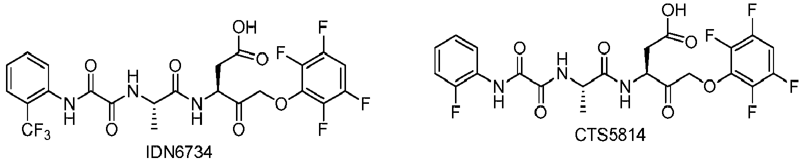 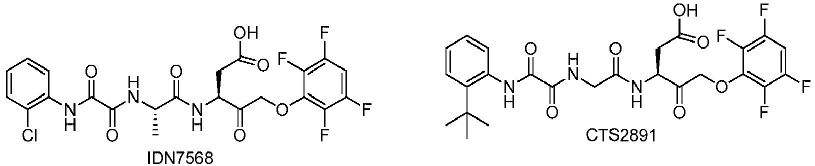 or a pharmaceutically acceptable derivative thereof.
44. The composition according to any of items 36-41, wherein the caspase inhibitor is selected from: 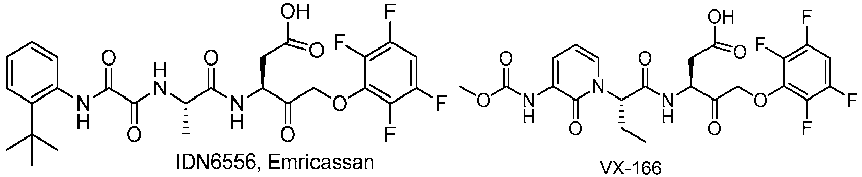 or a pharmaceutically acceptable derivative thereof.
45. The composition according to item 44, wherein the caspase inhibitor is selected from: 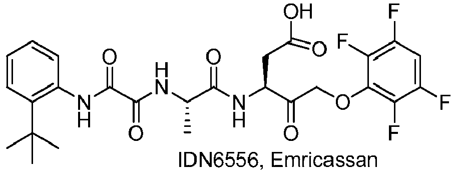 or a pharmaceutically acceptable derivative thereof.
46. The composition according to any of items 36-41, wherein the caspase inhibitor is selected from: 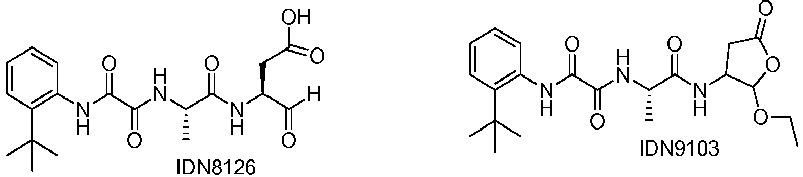 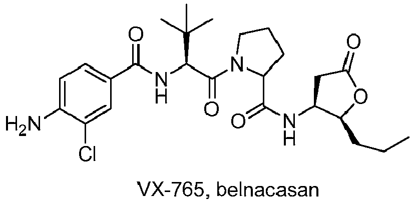 or a pharmaceutically acceptable derivative thereof.
47. The composition according to item 46, wherein the caspase inhibitor is selected from: 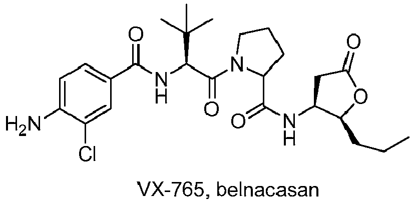 or a pharmaceutically acceptable derivative thereof.
48. The composition according to item 46, wherein the caspase inhibitor is selected from: 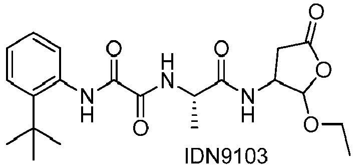 or a pharmaceutically acceptable derivative thereof.
49. A kit, comprising a caspase inhibitor and a second pharmacologically active substance, wherein the second pharmacologically active substance is for treating a complication or consequence of chronic liver disease.
50. The kit of item 49, wherein the caspase inhibitor is in a composition comprising the caspase inhibitor and a pharmaceutically acceptable excipient.
51. The kit of item 49, wherein the second pharmacologically active substance is in a composition comprising the caspase inhibitor and a pharmaceutically acceptable excipient.
52. The kit of any of items 49-51, wherein the caspase inhibitor and the second pharmacologically active substance are in a single composition.
53. The kit of any of items 49-51, wherein the caspase inhibitor and the second pharmacologically active substance are in two different compositions.
54. The kit of any of items 49-53, wherein the caspase inhibitor is selected from: 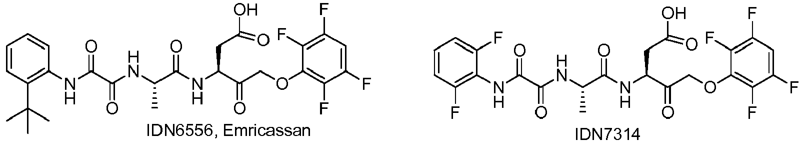 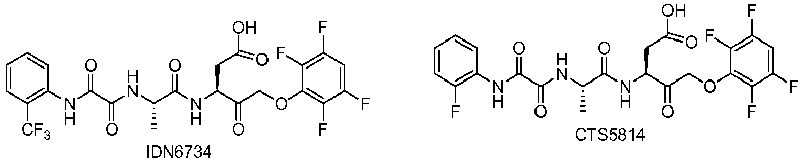 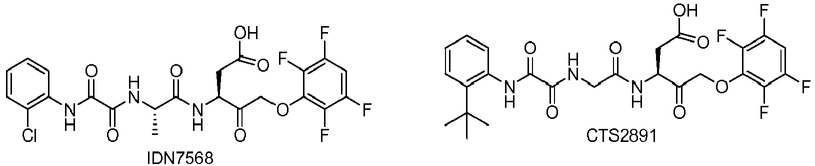 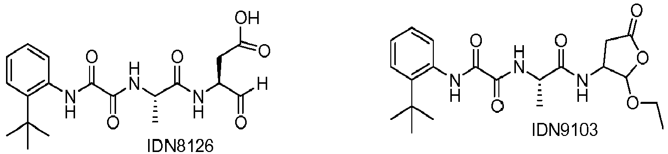 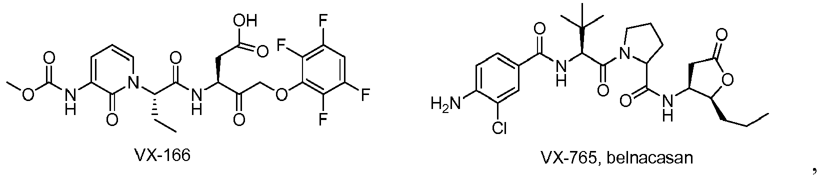 or a pharmaceutically acceptable derivative thereof.
55. A kit according to any of items 49-54, wherein the caspase inhibitor is selected from: 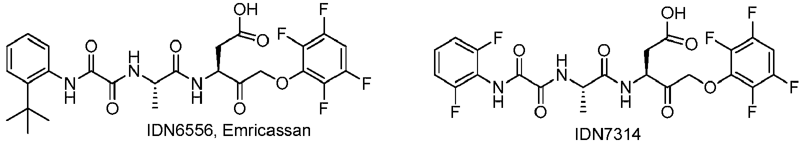 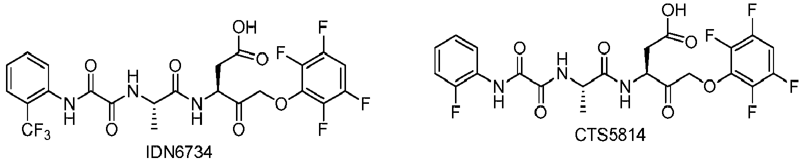 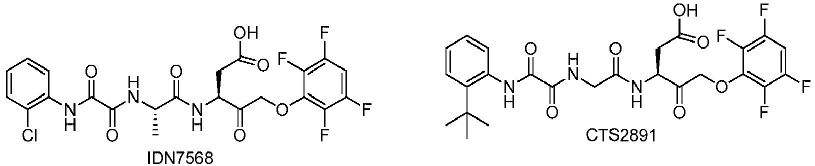 or a pharmaceutically acceptable derivative thereof.
56. A kit according to any of items 49-54, wherein the caspase inhibitor is selected from: 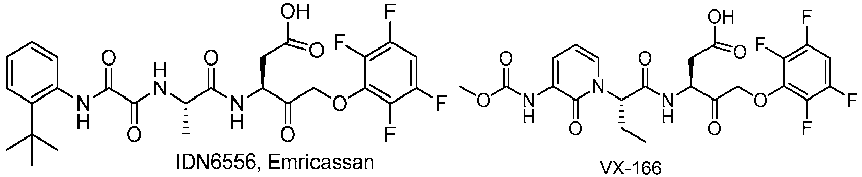 or a pharmaceutically acceptable derivative thereof.
57. The kit according to item 56, wherein the caspase inhibitor is: 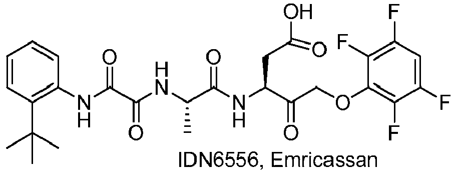 or a pharmaceutically acceptable derivative thereof.
58. A kit according to item 56, wherein the caspase inhibitor is: 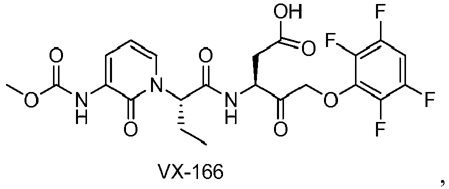 or a pharmaceutically acceptable derivative thereof.
59. A kit according to any of items 49-54, wherein the caspase inhibitor is selected from: 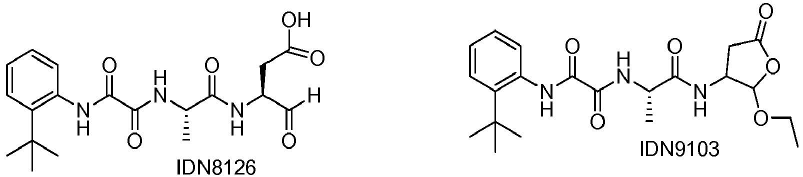 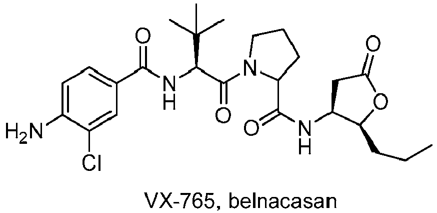 or a pharmaceutically acceptable derivative thereof.
60. A kit according to item 59, wherein the caspase inhibitor is: 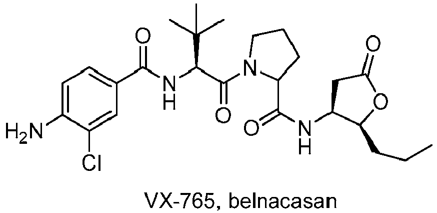 or a pharmaceutically acceptable derivative thereof.
61. A kit according to item 59, wherein the caspase inhibitor is: 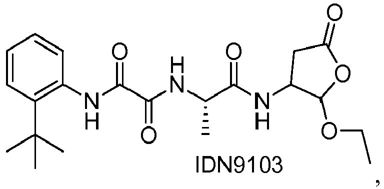 or a pharmaceutically acceptable derivative thereof.
62. A kit according to any of items 49-61, wherein the second pharmacologically active substance is selected from: Propranolol, Nadolol, Carvedilol, Simtuzumab (GS-6624), Sorafenib, Serelaxin (RLX030), Timolol, NCX-1000, Terlipressin, NGM282 and LUM001, and analogs or derivatives thereof.
63. A kit according to any of items 49-61, wherein the second pharmacologically active substance is a compound for the treatment of portal hypertension.
64. The kit of any of items 49-61, wherein the second pharmacologically active substance is a compound for the treatment of cirrhosis.
65. The method of any of items 1-35, further comprising administering a second pharmacologically active substance, wherein the second pharmacologically active substance is a compound for the treatment of a complication or consequence of chronic liver disease.
66. The method of item 65, wherein the second pharmacologically active substance is a compound for the treatment of cirrhosis or portal hypertension.
67. The method of item 65 or item 66, wherein the second pharmacologically active substance is administered simultaneously with the caspase inhibitor.
68. The method of item 65 or item 66, wherein the second pharmacologically active substance is administered sequentially in any order relative to the caspase inhibitor.
69. The method of any of items 65-68, wherein the second pharmacologically active substance is for the treatment of cirrhosis.
70. The method of item 69, wherein the compound is selected from among: wherein the second pharmacologically active substance is selected from: Simtuzumab (GS-6624), Sorafenib, Serelaxin (RLX030), Timolol, NCX-1000, Terlipressin, NGM282 and LUM001, and analogs or derivatives thereof.
71. The method of any of items 65-68, wherein the wherein the second pharmacologically active substance is for the treatment of portal hypertension.
72. The method of item 71, wherein the compound is selected from among: Propranolol, Nadolol, Carvedilol and Timolol.
73. The method of any of items 21-35 and 65-72, wherein the patient has failed therapy for portal hypertension.
74. The method of any of items 21-35 and 65-73, wherein the patient has failed therapy for liver cirrhosis.
75. A method of treatment, comprising selecting a subject with a chronic liver disease and elevated blood pressure in the liver, and administering a therapeutically effective amount of a caspase inhibitor to said subject, the amount being effective to mitigate chronic liver disease and/or lower the blood pressure in the liver.
76. The method of item 75, wherein the hepatic venous pressure gradient of the subject is greater than 5 mmHg.
77. The method of item 75, wherein the hepatic venous pressure gradient of the subject is greater than 10 mmHg.
78. The method of any of items 75-77, wherein the administration of the caspase inhibitor lowers the blood pressure in the liver.
79. The method of any of items 75-78, wherein the administration of the caspase inhibitor lowers the hepatic venous pressure gradient by 10% or more.
80. The method of any of items 75-79, wherein the administration of the caspase inhibitor lowers the hepatic venous pressure gradient by 5 mmHg or more.
81. The method of any of items 75-80, wherein the elevated blood pressure in the liver is determined by an elevated biomarker.
82. A method of treating a condition selected from among esophageal varices, ascites, hepatic encephalopathy, variceal hemorrhage and hepatocellular carcinoma, comprising administering, to a subject in need thereof, a therapeutically effective amount of a caspase inhibitor, whereby the condition is mitigated in the subject.
83. A method of treating liver cirrhosis and preventing portal hypertension comprising administering, to a subject in need thereof, a therapeutically effective amount of a caspase inhibitor, whereby the liver cirrhosis is mitigated and/or the risk of occurrence of portal hypertension is reduced.
84. The method of item 83, wherein the subject has an elevated level of alanine aminotransferase and wherein administering the caspase inhibitor lowers the level of alanine aminotransferase in the subject.

## Claims

1. A compound for use in a method of treating a complication of a chronic liver disease, wherein the compound is: 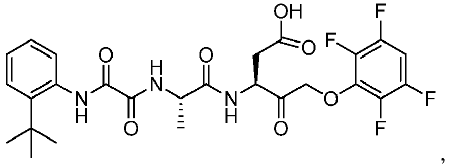 or a pharmaceutically acceptable salt thereof.

2. The compound for use of claim 1, wherein the complication of a chronic liver disease is selected from portal hypertension, esophageal varices, ascites, hepatic encephalopathy, variceal hemorrhage and hepatocellular carcinoma.

3. The compound for use of claim 2, wherein the complication of a chronic liver disease is portal hypertension.

4. A compound for use in a method of preventing decompensation in a liver cirrhosis subject, wherein the compound is: 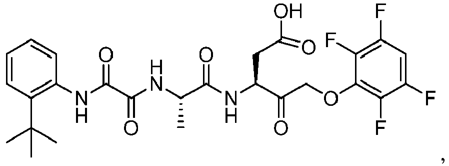 or a pharmaceutically acceptable salt thereof.

5. A compound for use in a method of preventing portal hypertension, wherein the compound is: 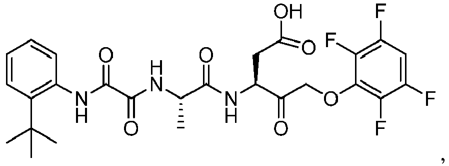 or a pharmaceutically acceptable salt thereof.

6. The compound for use of any one of claims 1 to 5, wherein the method comprises administering said compound in an amount from about 10 to about 150 mg per day.

7. The compound for use of claim 6, wherein the method comprises administering said compound in an amount from about 10 to about 50 mg per day.

8. The compound for use of any one of claims 1 to 7, wherein the method comprises administering said compound orally.

9. The compound for use of any one of claims 1 to 8, wherein method further comprises administering a second agent selected from propranolol, nadolol, carvedilol and timolol.

10. The compound for use of claim 9, wherein the second agent is propranolol.

11. The compound for use of claim 9, wherein the second agent is nadolol.

12. The compound for use of claim 9, wherein the second agent is carvedilol.

13. The compound for use of claim 9, wherein the second agent is timolol.
